# EUROPEAN PATENT APPLICATION

(11) **EP 2 550 909 A1**
(43) Date of publication of application: **30.01.2013**
(21) Application number: 11759095.0
(22) Date of filing: 16.02.2011
(51) Int. Cl.: A61B 1/04, A61B 1/00

(54) **ENDOSCOPE DEVICE**

(30) Priority: 24.03.2010 JP 2010067409
(71) Applicant: OLYMPUS CORPORATION, Shibuya-ku Tokyo 151-0072 (JP)
(72) Inventor: SHIDA, Hiromi, Tokyo 151-0072 (JP)
(74) Representative: Schicker, Silvia
(86) International application number: PCT/JP2011/053200
(87) International publication number: WO 2011/118287

(57) **Abstract**

To provide an endoscope apparatus with which an affected site can be treated with a treatment instrument while viewing tissue in the body cavity, including a region located at the rear of the treatment instrument. An endoscope apparatus 1 is employed, which is provided with an image generating portion 31 that generates an image of an subject A; an image-saving memory portion 32 that saves a real-time image; a forceps-region extracting portion 36 that extracts a forceps region, in which forceps exist, from the real-time image; an image-position aligning portion 35 that aligns positions of the saved image saved in the image-saving memory portion 32 and the real-time image; a forceps-region extracting portion 36 that extracts a region corresponding to the forceps region from the saved image saved in the image-saving memory portion 32; and an image combining portion 38 that combines an image of the region extracted by the forceps-region extracting portion 36 and the real-time image.

## Description

### {Technical Field}

The present invention relates to an endoscope apparatus.

### {Background Art}

In the related art, there is a known endoscope apparatus in which an inserted portion that is inserted into a body cavity is provided with an objective optical system that acquires an image of the body-cavity interior and a treatment instrument, such as forceps or the like (for example, see Patent Literature 1). Such an endoscope apparatus is configured so that an affected site can be treated with the treatment instrument while viewing the image of the body-cavity interior acquired by the objective optical system.

### {Citation List}

### {Patent Literature}

{PTL 1} Japanese Unexamined Patent Application, Publication No. 2002-34904

### {Summary of Invention}

### {Technical Problem}

However, with the endoscope apparatus disclosed in Patent Literature 1, there is a problem in that tissue in the body cavity cannot be observed in a region located at the rear of the treatment instrument with respect to the objective optical system because the treatment instrument interferes with the observation, which hinders the operation for treating the affected site.

The present invention has been conceived in light of the above-described circumstances, and an object thereof is to provide an endoscope apparatus with which an affected site can be treated with a treatment instrument while observing tissue in the body cavity, including a region located at the rear of the treatment instrument.

### {Solution to Problem}

In order to achieve the above-described object, the present invention employs the following solutions.
The present invention employs an endoscope apparatus provided with an image acquisition portion that acquires an image of an subject; an image saving portion that saves a current image acquired by the image acquisition portion; a treatment-instrument-region extracting portion that extracts a treatment-instrument region in which a treatment instrument exists from the current image acquired by the image acquisition portion; an image-position aligning portion that aligns positions of the saved image saved in the image saving portion and the current image acquired by the image acquisition portion; a treatment-instrument-corresponding-region extracting portion that extracts a region corresponding to the treatment-instrument region from the saved image saved in the image saving portion; and an image combining portion that combines an image of the region extracted by the treatment-instrument-corresponding-region extracting portion and the current image acquired by the image acquisition portion.

With the present invention, an image of the subject is acquired by the image acquisition portion, and the acquired image is saved in image saving portion. On the other hand, the treatment-instrument-region extracting portion extracts the treatment-instrument region, in which the treatment instrument (for example, biopsy forceps or the like) exists, from the current image acquired by the image acquisition portion. Then, the image-position aligning portion aligns positions of the saved image saved in the image saving portion and the current image acquired by the image acquisition portion, and the treatment-instrument-corresponding-region extracting portion extracts the region corresponding to the treatment-instrument region from the saved image saved in the image saving portion. The image of the region corresponding to the treatment-instrument region extracted in this way and the current image acquired by the image acquisition portion are combined by the image combining portion.

By doing so, even in the case in which a portion to be subjected to biopsy is hidden at the rear of the treatment instrument, it is possible to extract information about biological-subject region in the portion hidden at the rear of the treatment instrument (region corresponding to the treatment-instrument region) from a saved image, saved in advance in the image saving portion, and to display it by combining it with the current image acquired by the image acquisition portion. Accordingly, including a portion hidden at the rear of the treatment instrument, the positional relationship between the portion to be subjected to biopsy and the treatment instrument can be visually recognized in the image, which makes it possible to perform biopsy accurately.

The above-described invention may be provided with an image processing portion that generates an image in which the treatment-instrument region extracted by the treatment-instrument-region extracting portion is removed from the current image acquired by the image acquisition portion, wherein the image combining portion may combine the image of the region extracted by the treatment-instrument-corresponding-region extracting portion and the image generated by the image processing portion.

By doing so, the image processing portion generates an image in which the treatment-instrument region is removed from the current image, that is, a current image from which the treatment-instrument region is removed, thus including only the biological-subject portions. Then, the image combining portion combines the image extracted by the treatment-instrument-corresponding-region extracting portion and the image generated by the image processing portion. Accordingly, it is possible to generate a combined image from which the treatment-instrument region has been completely removed, which makes it possible to enhance observation precision for a portion to be subjected to biopsy.

In the above-described invention, the image combining portion may overlay positional information of the treatment-instrument region on the combined image.
By doing so, the positional information of the treatment-instrument region can be displayed overlaid on the combined image, which makes it possible for a user to easily ascertain the position of the treatment instrument and the position of the region in which the two images have been combined.

In the above-described invention, the image combining portion may overlay an outline of the treatment instrument as the positional information of the treatment-instrument region.
By doing so, the user can visually ascertain the biological-subject portion at the rear of the treatment instrument, and, also, because the position of the treatment instrument is displayed in the combined image in the form of the outline thereof, it is possible to easily ascertain the positional relationship between the portion to be subjected to biopsy and the treatment instrument.

In the above-described invention, the image combining portion may semi-transparently overlay the treatment instrument as the positional information of the treatment-instrument region.
By doing so, the user can visually ascertain the biological-subject portion at the rear of the treatment instrument, and, also, the position of the treatment instrument can be semi-transparently displayed in the combined image. Accordingly, three-dimensional information, such as the shape of the treatment instrument and so forth, can also be displayed, which makes it easier to ascertain the position and orientation of the treatment instrument, thus making it possible to perform biopsy more accurately.

The above-described invention may be provided with a characteristic-point detecting portion that detects characteristic points in the current image and the saved image, wherein the image-position aligning portion may align the positions of the current image and the saved image by using the characteristic points detected by the characteristic-point detecting portion.
By doing so, the characteristic-point detecting portion detects, for example, common characteristic points in the current image and the saved image, and the image-position aligning portion can align the positions of the current image and the saved image so that positions of these characteristic points are aligned. Accordingly, it is possible to enhance the precision in aligning the position of the current image and the saved image.

The above-described invention may be provided with a treatment-instrument detecting portion that detects the presence/absence of the treatment instrument in the current image, wherein, in the case in which the treatment-instrument detecting portion detects the treatment instrument, the treatment-instrument-region extracting portion may extract the treatment-instrument region from the current image.
By doing so, the treatment-instrument-region extracting portion extracts the treatment-instrument region from the current image acquired by the image acquisition portion, in the case in which the treatment-instrument detecting portion detects the treatment instrument. Accordingly, in the case in which the treatment instrument is not detected, processing by the treatment-instrument-corresponding-region extracting portion and image combining portion can be stopped, which makes it possible to reduce the amount of processing for the apparatus as a whole, thus enabling smooth endoscope observation.

In the above-described invention, the treatment-instrument detecting portion may detect the treatment instrument on the basis of color information of the current image.
Because the colors of the treatment instrument and the biological-subject tissue are different, whether or not the treatment instrument has entered the image can be detected by utilizing the color difference. Accordingly, by merely detecting color distribution in the image, the presence/absence of the treatment instrument can be easily detected.

The above-described invention may be provided with a treatment-instrument-position detecting portion that detects the position of the treatment instrument in the current image; and a movement-level calculating portion that calculates the movement level of the treatment instrument on the basis of the position of the treatment instrument detected by the treatment-instrument-position detecting portion, wherein, in the case in which the movement level calculated by the movement-level calculating portion is equal to or greater than a predetermined distance, the image saving portion may update an image to be saved.

By doing so, in the case in which the movement of the treatment instrument makes it possible to acquire an image of a location that has been hidden before, a new image of the location that has been hidden can be acquired by the image acquisition portion, and that image can be saved in the image saving portion. Accordingly, the saved image to be used for combining can be constantly updated, and the biological-subject information for the location hidden by the treatment instrument can be displayed by using images having as little time difference as possible.

In the above-described invention, the image saving portion may update an image to be saved at predetermined intervals.
By doing so, the saved image to be used for combining can be updated at the predetermined intervals, and the biological-subject information for the location hidden by the treatment instrument can be displayed by using images having as little time difference as possible. In addition, because it is not necessary to detect the position of the treatment instrument, the amount of processing can be reduced for the apparatus as a whole, which enables smooth endoscope observation.

The above-described invention may be provided with a region-dividing portion that divides the current image and the saved image into multiple regions; a gradation-value analyzing portion that calculates histograms of gradation values of the regions divided by the region-dividing portion for the current image and the saved image; and a gradation-value adjusting portion that adjusts gradation values of the individual regions in directions in which overlapping regions between the histograms of the current image calculated by the gradation-value analyzing portion and the histograms of the saved image are increased.

By doing so, the histograms for the current image acquired by the image acquisition portion can be made similar to the histograms for the saved image saved in the image saving portion. Accordingly, the gradation values of a region made darker due to the shadow of the treatment instrument and those of a region made brighter due to reflected light from the treatment instrument can be corrected in the current image. Accordingly, changes in the illumination conditions due to the positions of the treatment instrument can be suppressed, which makes it possible to display an image under uniform conditions.

The above-described invention may be provided with a region-dividing portion that divides the current image and the saved image into multiple regions; and a gradation-value detecting portion that detects gradation values of the regions divided by the region-dividing portion for the current image and the saved image, wherein, in the case in which a saturated region in which the gradation values have saturated exists in the current image, the image combining portion may replace an image of the saturated region with the saved image.

By doing so, the saturated region, in which saturation has occurred, in the current image acquired by the image acquisition portion can be displayed by replacing it with the saved image saved in the image saving portion. Accordingly, even for a region for which information about the form thereof is lost due to saturation, because the information about the form can be acquired by switching images, the state of a biological subject can be ascertained regardless of the illumination conditions.

The above-described invention may be provided with a characteristic-point detecting portion that detects a characteristic point in the current image; and a characteristic-point searching portion that searches for the characteristic point detected by the characteristic-point detecting portion in a plurality of saved images saved in the image saving portion, wherein the treatment-instrument-corresponding-region extracting portion may extract a region corresponding to the treatment-instrument region from the saved image in which the characteristic point has been searched for by the characteristic-point searching portion.

By doing so, even in the case in which the characteristic points of the current image are not found in the most recent saved image saved in the image saving portion, a saved image having the characteristic points is searched for among the plurality of saved saved images, and the treatment-instrument region can be extracted from that saved image and combined with the current image. Accordingly, biological-subject information of the treatment-instrument region can be displayed even in the case in which the image has considerably changed due to considerable movement of the tip of the endoscope or the case in which the angle of view has changed.

In the above-described invention, the treatment-instrument-corresponding-region extracting portion may enlarge or reduce the saved image and may extract the region corresponding to the treatment-instrument region from the enlarged or reduced saved image.
By doing so, the region corresponding to the treatment-instrument region can be extracted from the enlarged or reduced saved image and combined with the current image acquired by the image acquisition portion, even in the case in which the size of the saved image and the size of the current image are different.

The above-described invention may be provided with a characteristic-point detecting portion that detects a plurality of characteristic points in the current image and the saved image; and an enlargement-ratio setting portion that sets an enlargement ratio for the saved image relative to the current image on the basis of distances between the plurality of characteristic points in the current image and the saved image detected by the characteristic-point detecting portion, wherein the treatment-instrument-corresponding-region extracting portion may enlarge or reduce the saved image by the enlargement ratio set by the enlargement-ratio setting portion.

By doing so, by comparing the distance between the plurality of characteristic points in the current image and the distance between the plurality of corresponding characteristic points in the saved image, it is possible to set the enlargement ratio for the saved image relative to the current image. Accordingly, the region corresponding to the treatment-instrument region can be extracted from the saved image enlarged or reduced by the set enlargement ratio and combined with the current image acquired by the image acquisition portion, even in the case in which the size of the saved image and the size of the current image are different.

### {Advantageous Effects of Invention}

The present invention affords an advantage in that a affected site can be treated with a treatment instrument while observing tissue in the body cavity, including a region located at the rear of the treatment instrument.

### {Brief Description of Drawings}

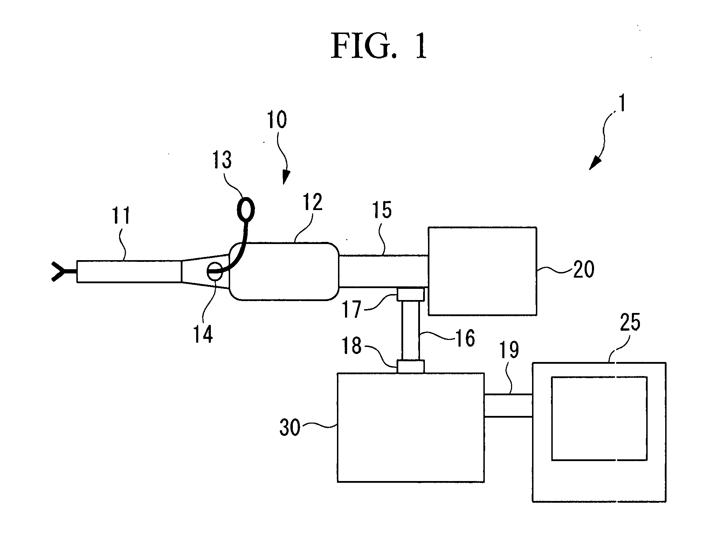

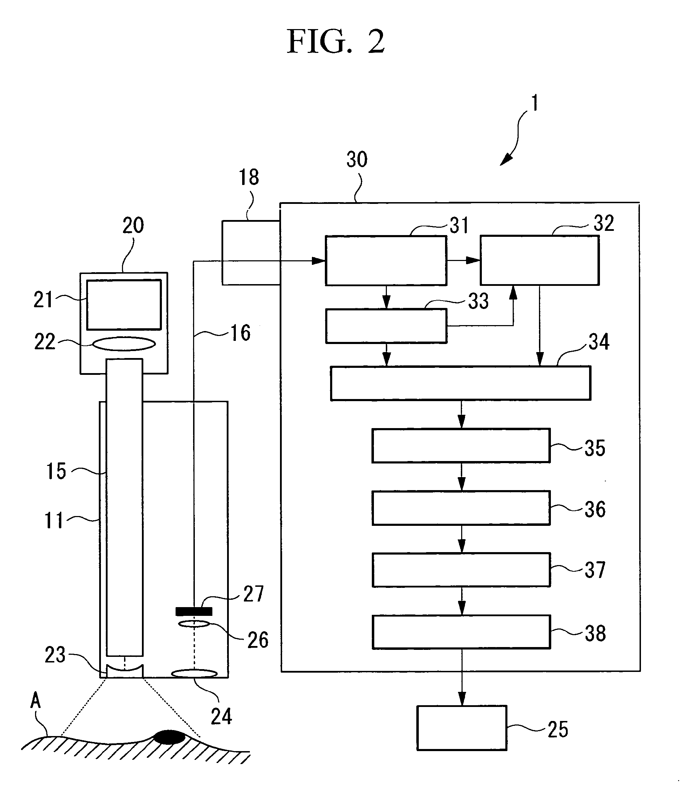

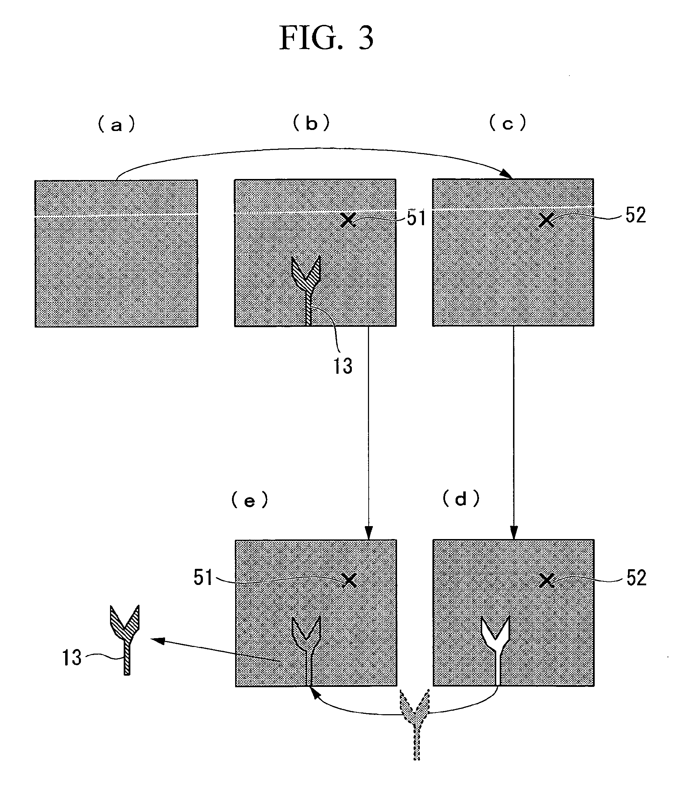

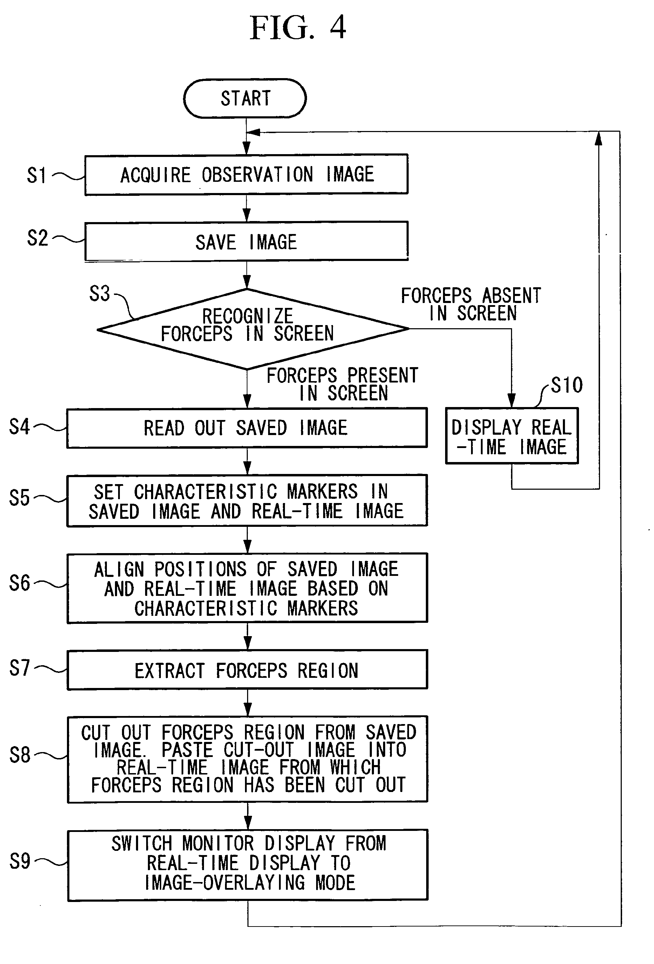

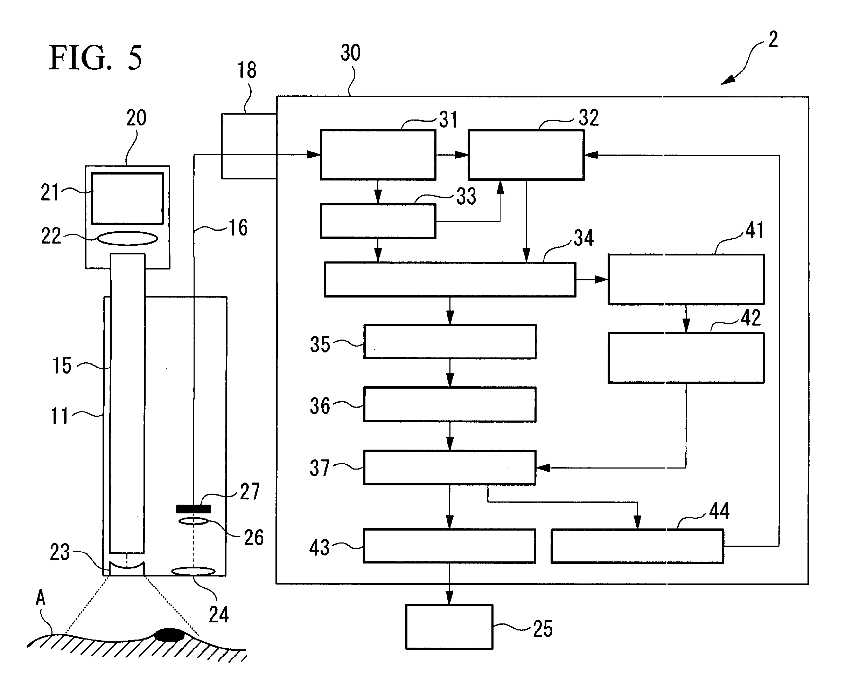

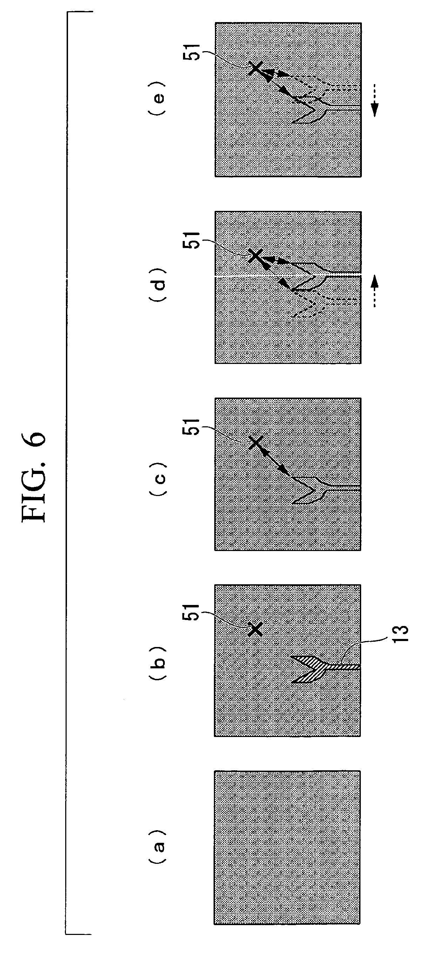

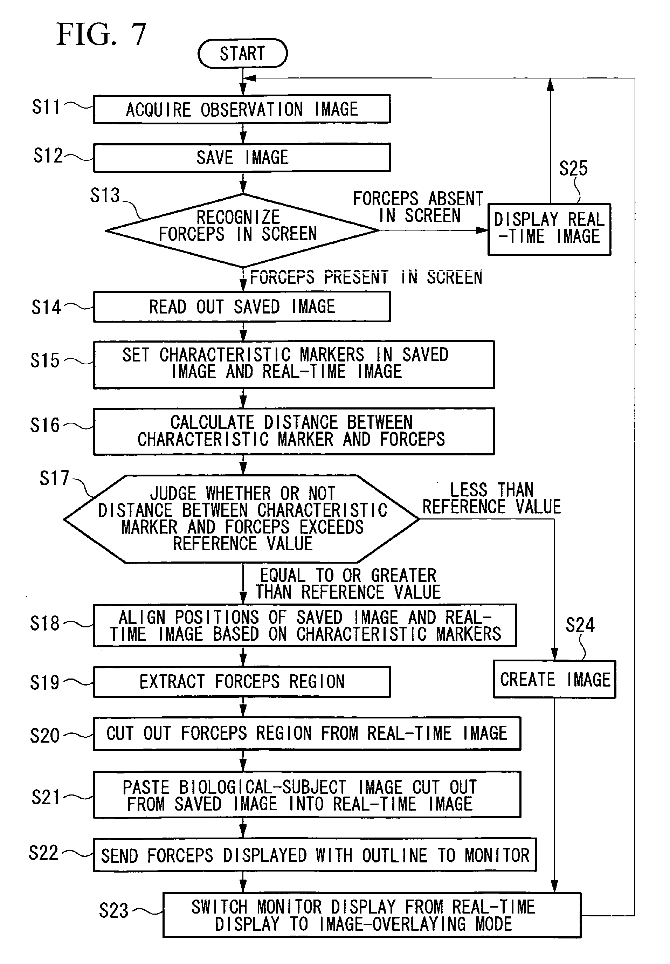

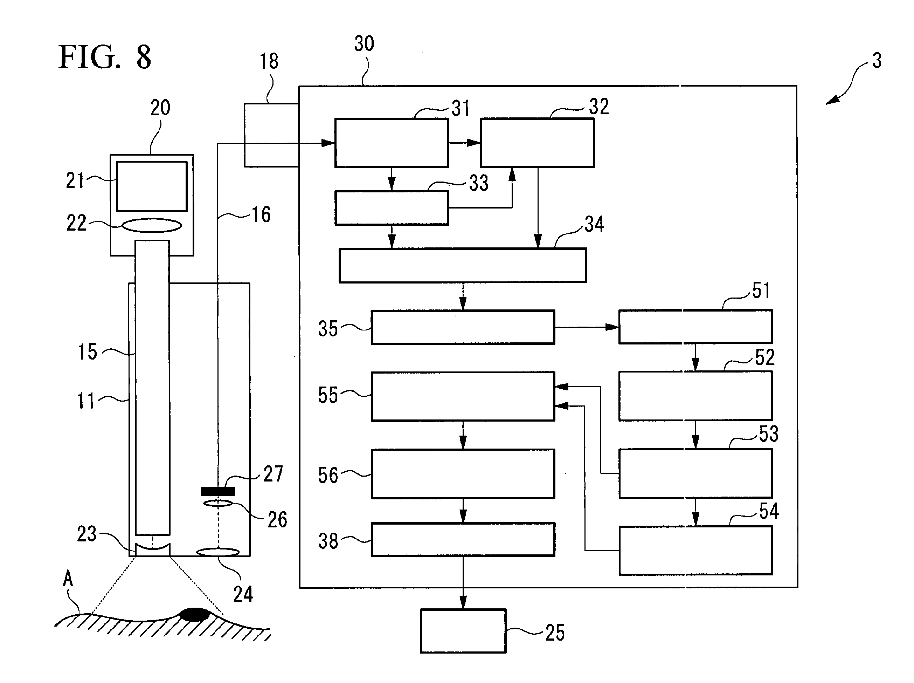

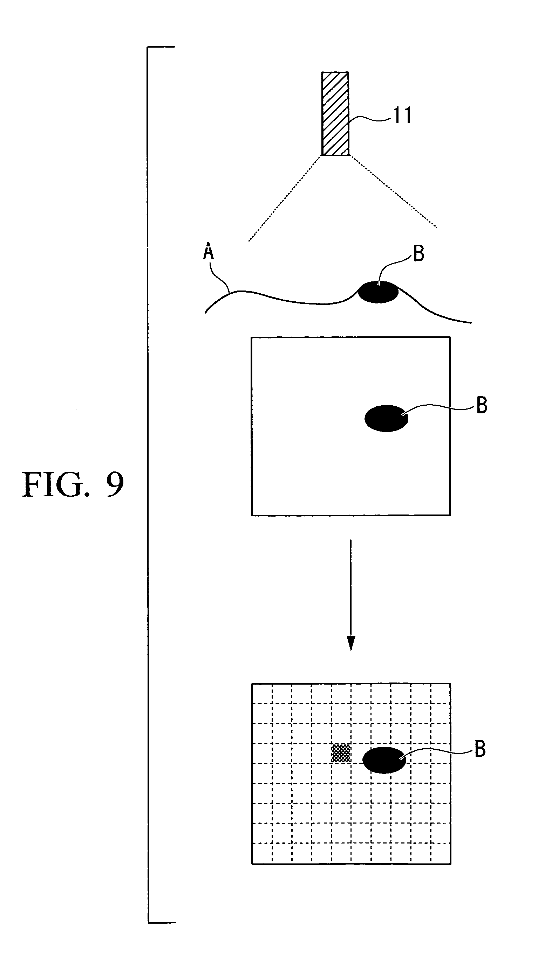

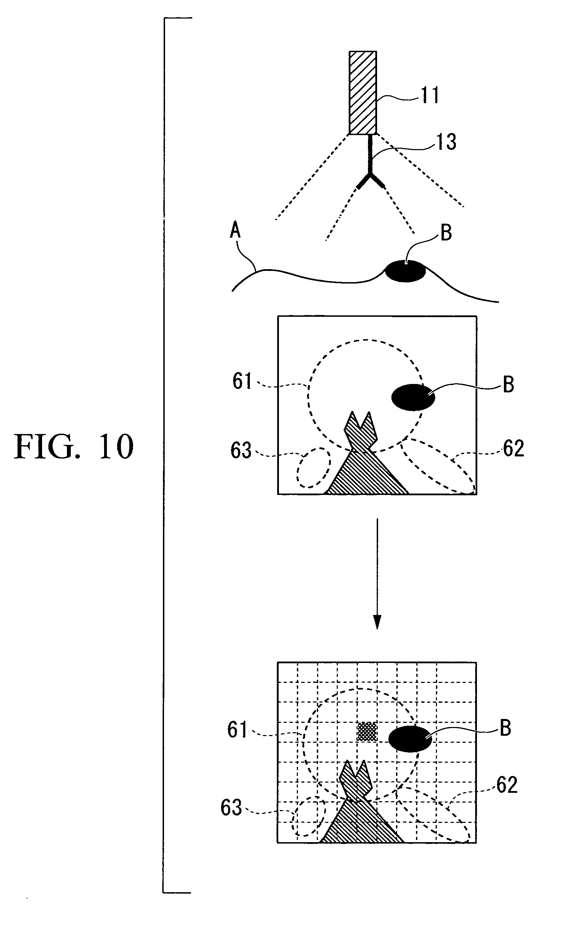

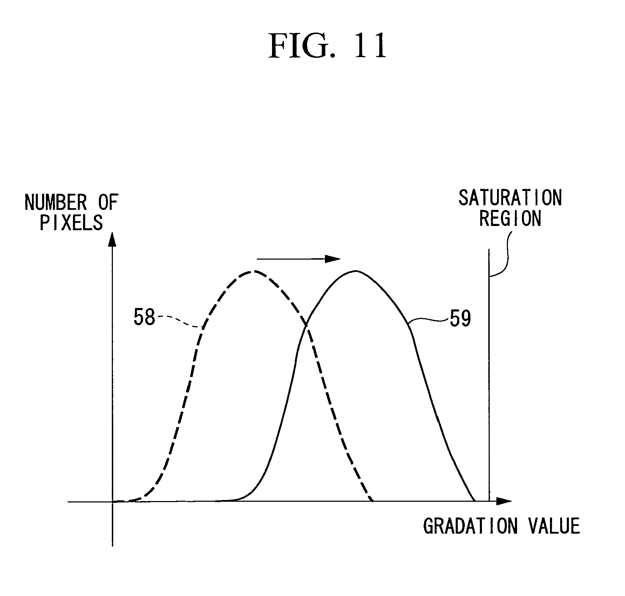

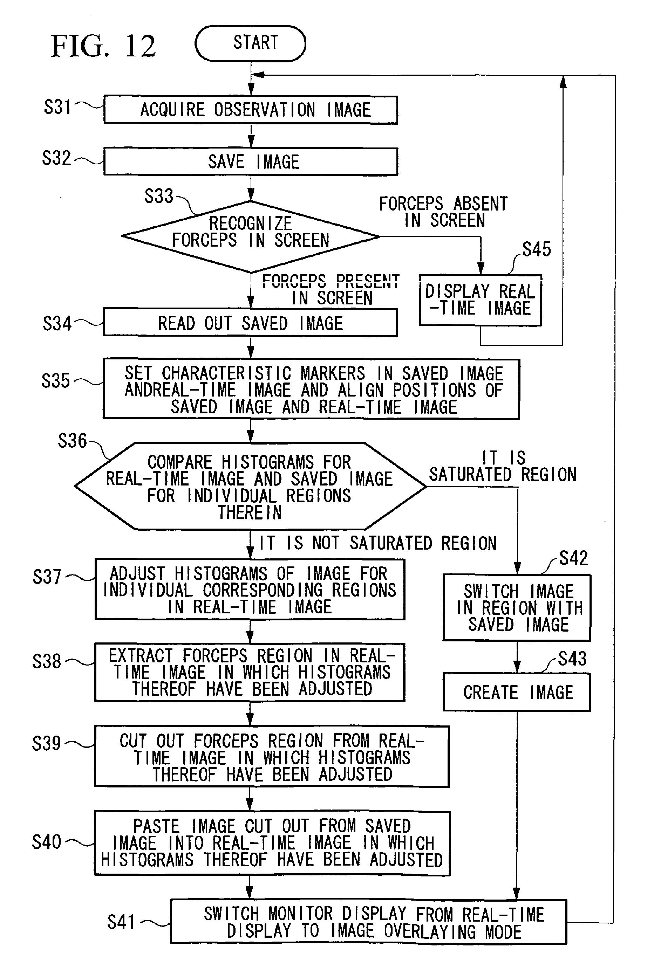

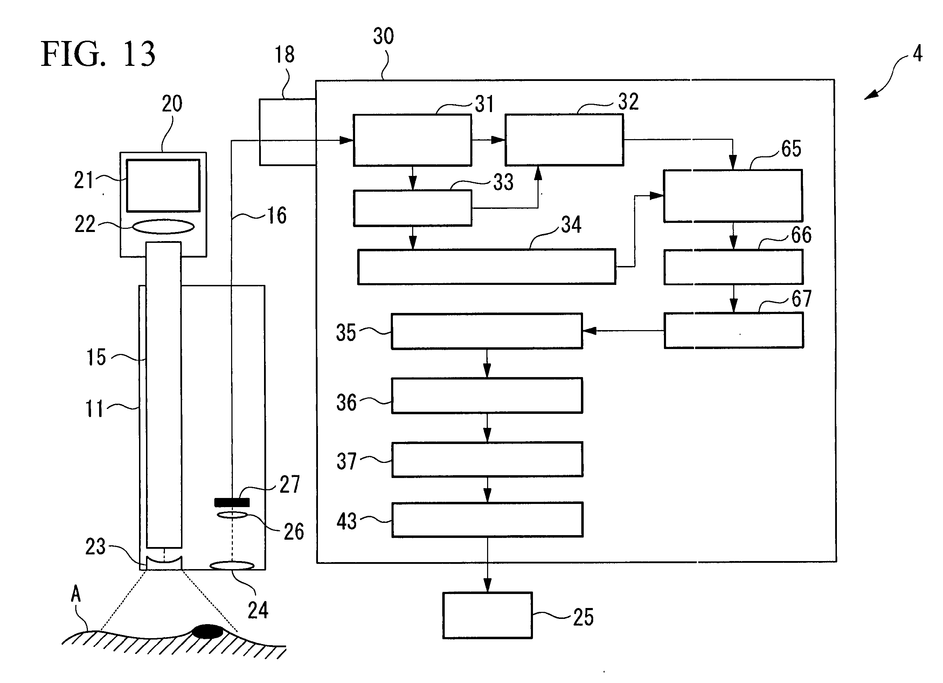

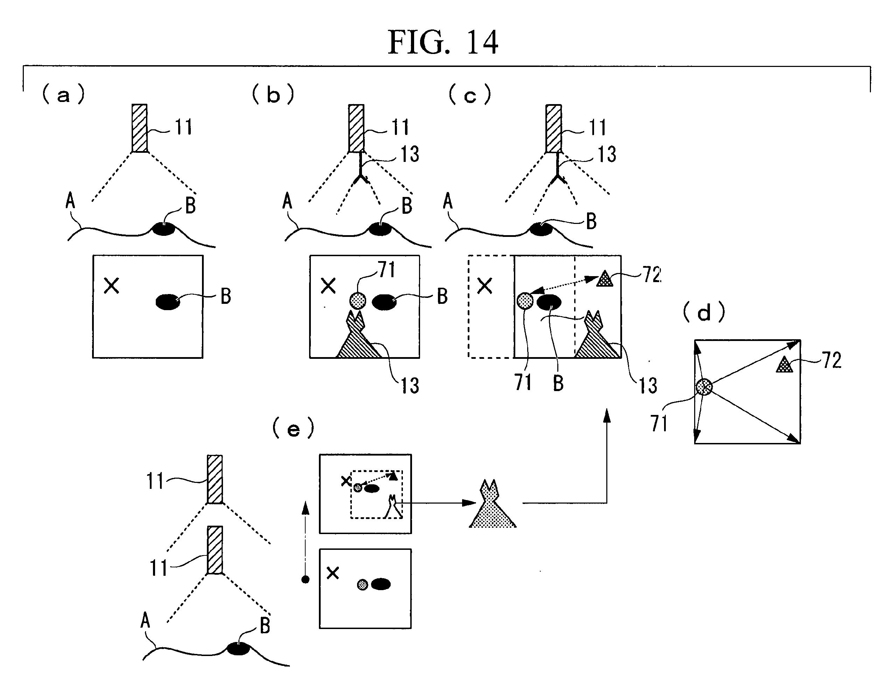

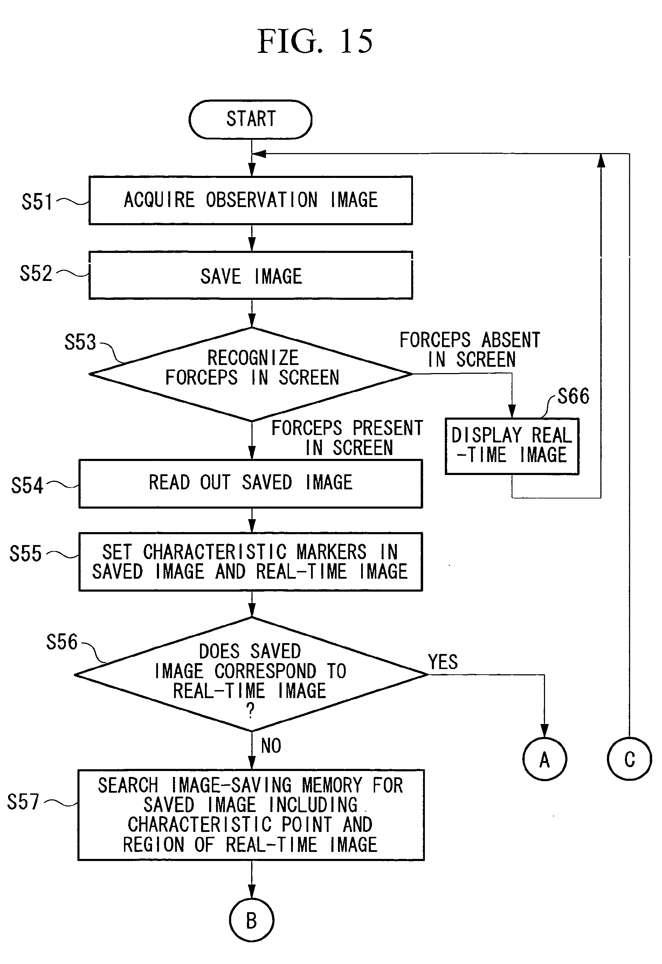

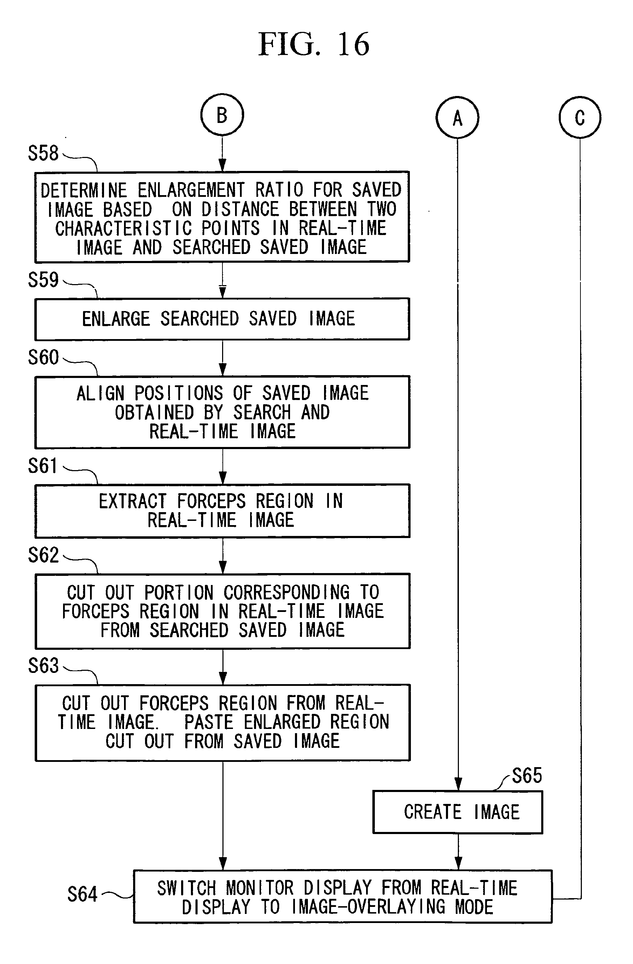

{Fig. 1} Fig. 1 is a diagram showing the overall configuration of an endoscope apparatus according to the individual embodiments of the present invention.
{Fig. 2} Fig. 2 is a functional block diagram of an endoscope apparatus according to a first embodiment of the present invention.
{Fig. 3} Fig. 3 is a diagram showing example images for individual each process executed by the endoscope apparatus in Fig. 2, in which Fig. 3(a) shows a real-time image before insertion of forceps; Fig. 3(b) shows a real-time image after insertion of the forceps; Fig. 3(c) shows an image before insertion of the forceps, saved in an image-saving memory portion 32; Fig. 3(d) shows an image in which a portion corresponding to a forceps region is cut out from a saved image; and Fig. 3(e) shows an image in which the image in Fig. 3(b) and the image cut out in Fig. 3(d) are combined.
{Fig. 4} Fig. 4 is a flowchart showing the processing executed by the endoscope apparatus in Fig. 2.
{Fig. 5} Fig. 5 is a functional block diagram of an endoscope apparatus according to a second embodiment of the present invention.
{Fig. 6} Fig. 6 is a diagram showing example images for each process executed by the endoscope apparatus in Fig. 5, in which Fig. 6(a) shows a real-time image before insertion of forceps; Fig. 6(b) shows a real-time image after insertion of the forceps; Fig. 6(c) shows an image in which a saved image is combined with a forceps region in the real-time image; and
Fig. 6(d) and Fig. 6(e) show images for the cases in which the forceps are moved in the image in Fig. 6(c).
{Fig. 7} Fig. 7 is a flowchart showing the processing executed by the endoscope apparatus in Fig. 5.
{Fig. 8} Fig. 8 is a functional block diagram of an endoscope apparatus according to a third embodiment of the present invention.
{Fig. 9} Fig. 9 shows an example image in which a saved image is divided into multiple regions.
{Fig. 10} Fig. 10 shows an example image in which a real-time image is divided into multiple regions.
{Fig. 11} Fig. 11 shows histograms of gradation values for a real-time image and a saved image.
{Fig. 12} Fig. 12 is a flowchart showing the processing executed by the endoscope apparatus in Fig. 8.
{Fig. 13} Fig. 13 is a functional block diagram of an endoscope apparatus according to a fourth embodiment of the present invention.
{Fig. 14} Fig. 14 is a diagram showing example images for each process executed by the endoscope apparatus in Fig. 13, in which Fig. 14(a) shows a real-time image before insertion of forceps; Fig. 14(b) shows a real-time image after insertion of the forceps; Fig. 14(c) shows a real-time image for the case in which an image-capturing position is changed; Fig. 14(d) shows an example image for explaining a method of detecting the position of a characteristic point; and Fig. 14(e) shows an example image for explaining processing for enlarging/reducing a saved image.
{Fig. 15} Fig. 15 is a flowchart showing the processing executed by the endoscope apparatus in Fig. 13.
{Fig. 16} Fig. 16 is a flowchart showing the processing executed by the endoscope apparatus in Fig. 13.

### {Description of Embodiments}

### First Embodiment

An endoscope apparatus 1 according to a first embodiment of the present invention will be described below with reference to the drawings.
As shown in Fig. 1, the endoscope apparatus 1 according to this embodiment is provided with an endoscope 10 that acquires an image of an subject, a light-source device 20 that emits illumination light into the endoscope 10, a control unit 30 that processes the image acquired by the endoscope 10, and a monitor 25 that displays the image processed by the control unit 30.

The endoscope 10 is provided with a long, thin inserted portion 11 that is inserted into a body cavity, a holding portion 12 provided at the basal end of the inserted portion 11, and a forceps inlet 14 provided between the inserted portion 11 and the holding portion 12, into which a treatment instrument, such as forceps 13 or the like, is inserted.

The endoscope 10 (the basal end of the holding portion 12) and the light-source device 20 are connected by a light-guide cable 15 that guides the illumination light from the light-source device 20.
The endoscope 10 (the basal end of the holding portion 12) and the control unit 30 are connected by an image transmission cable 16 that transmits image data acquired by the endoscope 10 via the light-guide cable 15.

The light-guide cable 15 and the image transmission cable 16 are connected via an electrical connector 17.
The image transmission cable 16 and the control unit 30 are connected via a connecting connector 18.
The control unit 30 and the monitor 25 are connected with a monitor cable 19 that transmits image data processed by the control unit 30.

With this configuration, the illumination light emitted from the light-source device 20 is optically guided by the light-guide cable 15 to be radiated onto an subject in the body cavity from the tip of the endoscope 10. Then, an image of the subject is acquired by the endoscope 10, and image data thereof are sent to the control unit 30 via the image transmission cable 16. The image data sent thereto are subjected to image processing at the control unit 30 and are subsequently transmitted to the monitor 25 via the monitor cable 19 to be displayed on a monitor screen.

Next, the detailed configuration of the endoscope apparatus 1 of this embodiment will be described by using Fig. 2.
As shown in Fig. 2, a xenon lamp (Xe lamp) 21 and a relay lens 22 are installed inside the light-source device 20. Light emitted from the Xe lamp 21 is optically guided by the light-guide cable 15 in the endoscope 10 via the relay lens 22 and is radiated onto the subject A by means of an illumination optical system 23 disposed at the tip of the endoscope 10. Reflected light from the subject A enters an image-capturing optical system 24 disposed at the tip of the endoscope 10.

The reflected light that has entered the image-capturing optical system 24 is detected by a color CCD 27 installed at a stage subsequent to the image-capturing optical system 24 via a relay lens 26 and is converted to image data. The image data converted by the color CCD 27 are sent to an image generating portion 31 in the control unit 30 via the image transmission cable 16.

As shown in Fig. 2, the control unit 30 is provided with, as its functions, the image generating portion (image acquisition portion) 31, an image-saving memory portion (image saving portion) 32, a forceps detecting portion (treatment-instrument detecting portion) 33, an intra-image-characteristic-marker recognition portion (characteristic-point detecting portion) 34, an image-position aligning portion 35, a forceps-region extracting portion (treatment-instrument-region extracting portion, treatment-instrument-corresponding-region extracting portion) 36, a forceps-region image processing portion (image processing portion) 37, and an image combining portion 38.

The image generating portion 31 generates an image of the subject A from the image data converted by the color CCD 27. The image generated at the image generating portion 31 is sent to the image-saving memory portion 32 and the forceps detecting portion 33.

The image-saving memory portion 32 sequentially saves images sent thereto. The image-saving memory portion 32 saves images for a certain duration, for example, about 5 seconds, starting from several seconds before reaching the current time. As will be described later, the endoscope apparatus 1 of this embodiment acquires, from saved images saved in this image-saving memory portion 32, an image before the forceps 13 are detected, and, after the forceps 13 are detected, biological-subject information behind the forceps is displayed by pasting a portion of the saved image corresponding to a forceps portion.

The forceps detecting portion 33 judges, by means of color recognition or the like, whether or not the forceps 13 exist in an image on the basis of the image sent thereto. Here, in order to observe a biological subject, an observation screen of an endoscope apparatus is normally displayed in reddish colors, whereas the forceps 13 have silver or white. Therefore, if the forceps 13 exist in the observation screen, which is normally displayed in reddish colors, the presence of the forceps 13 can be detected by means of color because the forceps 13 have silver or white, which is different from the color of the biological subject.

If the forceps detecting portion 33 judges that the forceps 13 do not exist in the image, the forceps detecting portion 33 sends the image generated by the image generating portion 31 to the monitor 25 without modification so as to display a real-time image (current image) on the monitor 25, and sequentially saves new images in the image-saving memory portion 32.

On the other hand, if the forceps detecting portion 33 judges that the forceps 13 exist in the image, the forceps detecting portion 33 stops writing new images in the image-saving memory portion 32 and also outputs to the image-saving memory portion 32 an instruction for retaining image immediately before the forceps 13 were recognized. In this case, the saved image retained at the image-saving memory portion 32 is sent to the intra-image-characteristic-marker recognition portion 34 from the image-saving memory portion 32. Tn addition, the forceps detecting portion 33 sends a real-time image at that time to the intra-image-characteristic-marker recognition portion 34.

With regard to the real-time image and the saved image, the intra-image-characteristic-marker recognition portion 34 identifies portions that serve as characteristic markers in the images by selecting, for example, points where the luminance is higher than the surroundings, points where the color is different, and so forth, in the respective images.

A specific method of identifying the characteristic points will be described by using examples shown in Figs. 3(a) to 3(e). Fig. 3(a) shows a real-time image before insertion of the forceps, Fig. 3(b) shows a real-time image after insertion of the forceps, Fig. 3(c) shows an image before the insertion of the forceps, saved in the image-saving memory portion 32, Fig. 3(d) shows an image in which a portion corresponding to a forceps region is cut out from the saved image, and Fig. 3(e) shows an image in which the image in Fig. 3(b) and the cut-out image in Fig. 3(d) are combined.

In the real-time image shown in Fig. 3(b) and the saved image shown in Fig. 3(c), × marks 51 and 52 are the characteristic-marker portions identified to be the characteristic points. The real-time image and the saved image in which the characteristic markers are identified in this way are sent to the image-position aligning portion 35 from the intra-image-characteristic-marker recognition portion 34.

The image-position aligning portion 35 aligns positions of the real-time image and the saved image on the basis of the characteristic-marker information. In the examples shown in Fig. 3(a) to 3(e), the × marks 51 and 52 are added to the real-time image and the saved image, respectively, and positions of the real-time image and the saved image are aligned so that the positions of the × marks 51 and 52 coincide with each other.

Note that, in order to simplify the description, the description herein is given assuming the case in which the screen is moved horizontally; however, in the case in which, for example, the saved image and the real-time image are rotated, two characteristic points should be set for position alignment, and the positions should be aligned so that these two points coincide with each other.

The real-time image and the saved image whose positions have been aligned at the image-position aligning portion 35 are sent to the forceps-region extracting portion 36.
With regard to the real-time image, the forceps-region extracting portion 36 extracts the outline of the forceps 13 by utilizing the color difference, as with the forceps detecting portion 33. Specifically, the forceps-region extracting portion 36 extracts the outline of the forceps region (treatment-instrument region) by distinguishing a border between the forceps 13 and a biological-subject portion on the basis of the color difference between the forceps 13 and the biological subject.

In addition, the forceps-region extracting portion 36 extracts a portion corresponding to the forceps region in the saved image on the basis of the information about the extracted forceps region of the real-time image. This is preparation for subsequently cutting out an image region corresponding to the forceps 13 in the real-time image from the saved image.

The forceps-region image processing portion 37 performs an image-cut-out operation on the basis of the images and information about the forceps region sent thereto from the forceps-region extracting portion 36. As shown in Fig. 3(e), with regard to the real-time image, the image inside the forceps outline is cut out, thereby leaving only the biological-subject portions. The image of the forceps 13 that is cut out here is not used because it makes the image of the biological subject invisible, hiding biological-subject information.

As shown in Fig. 3(d), with regard to the saved image, a portion corresponding to the forceps region in the real-time image, which is extracted at the forceps-region extracting portion 36, is cut out. In other words, a portion that is not visible in the real-time image because it is behind the forceps 13 is taken out from the saved image. The real-time image from which the forceps 13 are removed and the saved image which is a cutout showing the region corresponding to the portion that was not visible because it is behind the forceps 13, generated in this way, are sent to the image combining portion 38.

As shown in Fig. 3(e), the image combining portion 38 performs image combining between the real-time image and the saved image by combining the two images sent thereto from the forceps-region image processing portion 37. In this way, a combined image in which the portion behind the forceps 13 is made visible is created by removing the biological-subject information, which is not visible because it is behind the forceps 13, from the saved image and by pasting it into the portion in the real-time image from which the forceps 13 have been removed. In addition, the image combining portion 38 also performs outline display by using a border portion where the two images are combined as the outline of the forceps 13. Note that the outline display may be performed by displaying several pixels left at the outline portion of the forceps 13.

By sending the image combined as described above to the monitor 25, the combined image in which the biological-subject image is pasted inside the outline, which indicates the forceps region, is displayed on the monitor screen.
Note that, although the portion behind the forceps is displayed in this embodiment by processing images after position alignment, it suffices to finally display the information about the biological-subject behind the forceps. Therefore, the order of processing may be such that, for example, positions of the real-time image and the saved image are aligned after extracting the forceps region and processing the images.

The operation of the endoscope apparatus 1 having the above-described configuration will be described in accordance with a flowchart shown in Fig. 4. Fig. 4 shows a flowchart indicating steps from finding a diseased portion by means of endoscope observation to performing biopsy, employing the endoscope apparatus 1 of this embodiment.

First, once observation is started using the endoscope apparatus 1 of this embodiment, the illumination light from the laser light source 20 is radiated onto the subject A, and the reflected light from the subject A is detected by the color CCD 27 to be converted to image data. An image is generated at the image generating portion 31 on the basis of the image data, and the generated image of the subject A is displayed on the monitor 25 (Step S1).

The image is displayed on the monitor 25 as shown in Fig. 3(a), and a surgeon observes this image, searching for a biopsy target portion. At this time, images up to, for example, 5 seconds before the time of observation, are constantly saved in the image-saving memory portion 32 (Step S2).

In this state, the forceps detecting portion 33 judges whether or not the forceps 13 exist in an image (Step S3).
If the forceps 13 are not present in the image in Step S3, the real-time image is displayed on the monitor 25 without modification (Step S10).

In the case in which the biopsy target site is found within the observation viewing field in the real-time image, the forceps 13 are inserted into the forceps inlet 14 in the endoscope 10 so that the forceps 13 are inserted through to the tip of the inserted portion 11. By doing so, the forceps 13 appear within the observation viewing field, as shown in Fig. 3(b). The forceps detecting portion 33 recognizes the presence/absence of the forceps 13 in the observation viewing field by means of a color change or the like.

In the case in which the forceps 13 exists in the image in Step S3, the saved image immediately before the appearance of the forceps 13 is read out from the image-saving memory portion 32 (Step S4).
Next, the intra-image-characteristic-marker recognition portion 34 sets the characteristic markers in the read-out saved image and the real-time image by utilizing points where the luminance is higher than the surroundings, points where the color is different, and so forth (Step S5).

Next, the image-position aligning portion 35 aligns the positions of the saved image and the real-time image on the basis of the set characteristic markers (Step S6).
Next, the forceps-region extracting portion 36 extracts the outline of the forceps 13 and the forceps region from the real-time image on the basis of the color difference between the forceps 13 and the biological subject (Step S7). This forceps region is also used when performing the cut-out operation on the saved image.

Next, the forceps-region image processing portion 37 cuts out the forceps region from the real-time image, leaving the remaining biological-subject portions, and also cut out the portion corresponding to the forceps region from the saved image (Step S8). Then, the image combining portion 38 pastes the biological-subject information cut out from the saved image into the image having the remaining biological-subject information of the real-time image. At this time, outline display is also performed because the boundary portion where the two images are combined forms the outline of the forceps 13.

Next, the image display is switched from the real-time display to an image-overlaying mode (Step S9). When the display is switched to the image-overlaying mode, the forceps 13 are switched to the outline display, which makes the portion hidden by the forceps 13 visible, as shown in Fig. 3(e). By doing so, the surgeon can advance the forceps 13 toward the biopsy target site and perform biopsy, while viewing the screen in which the portion that was hidden by the forceps 13 has become visible.

Note that, because the forceps 13 disappear from the screen when the biopsy is completed, the absence of the forceps 13 is recognized, and the screen display is switched from the image-overlaying mode to the real-time display.

As described above, with the endoscope apparatus 1 according to this embodiment, even in the case in which a site to be subjected to biopsy is hidden at the rear of the forceps 13, it is possible to extract information about the biological-subject region in a portion hidden at the rear of the forceps 13 (region corresponding to the forceps region) from a saved image, which is saved in advance in the image-saving memory portion 32, and to display it by combining it with the real-time image. Accordingly, even for a portion that is hidden at the rear of the forceps 13, the positional relationship between a portion to be subjected to biopsy and the forceps 13 can be visually recognized in the image, which makes it possible to perform the biopsy accurately.

In addition, the forceps-region image processing portion 37 generates an image in which the forceps region is removed from the real-time image, that is, a real-time image from which the region of the forceps 13 is removed, thus including only the biological-subject portions. Then, the image combining portion 38 combines the image extracted by the forceps-region extracting portion 36 and the image generated by the forceps-region image processing portion 37. By doing so, it is possible to generate a combined image from which the region of the forceps 13 has been completely removed, which makes it possible to enhance observation precision for a portion to be subjected to biopsy.

In addition, by overlaying the outline of the forceps 13 as the positional information for the forceps region, the image combining portion 38 allows a user to visually ascertain the biological-subject portion at the rear of the forceps 13, and, also, because the position of the forceps 13 is displayed in the combined image in the form of the outline thereof, it is possible to easily ascertain the positional relationship between the portion to be subjected to biopsy and the forceps.

In addition, the intra-image-characteristic-marker recognition portion 34 detects, for example, common characteristic points in the real-time image and the saved image; the image-position aligning portion 35 aligns the positions of the real-time image and the saved image by using these characteristic points; and, by doing so, it is possible to enhance the precision in aligning the positions of the real-time image and the saved image.

In addition, by extracting the forceps region from the real-time image by means of the forceps-region extracting portion 36 in the case in which the forceps detecting portion 33 detects the forceps 13, the processing by the forceps-region extracting portion 36 and the image combining portion 38 can be stopped if the forceps 13 are not detected, which makes it possible to reduce the amount of processing for the apparatus as a whole, thus enabling smooth endoscope observation.

In addition, because the colors of the forceps 13 and the biological-subject tissue are different, by causing the forceps detecting portion 33 to detect the forceps 13 on the basis of the color information in the real-time image, it is possible to detect whether or not the forceps 13 have entered the image by utilizing this color difference. Accordingly, the presence/absence of the forceps 13 can easily be detected merely by detecting the color distribution in the image.

### {Second Embodiment}

Next, an endoscope apparatus 2 according to a second embodiment of the present invention will be described by using Figs. 5 to 7.
Fig. 5 is a functional block diagram of the endoscope apparatus 2 according to the embodiment, and the configuration thereof is the same as that of the endoscope apparatus 1 according to the first embodiment, except for the processing in the control unit 30.

The endoscope apparatus 2 according to the second embodiment is the same as the endoscope apparatus 1 according to the first embodiment described above in terms of the processing up to the generation of the observation image.
Here, with the endoscope apparatus 1 according to the first embodiment, once the saved image to be used for creating the combined image is determined and retained, thereafter, the forceps region in the real-time image is created by using the retained saved image. Because of this, if the amount of time during which the forceps 13 are present in the image increases, information in the retained saved image becomes outdated with respect to that of the real-time image. Therefore, when the manner in which the biological-subject portion appear changes due to the influence of illumination or when the state of the diseased portion changes by the minute, because the information of the retained saved image is old, a mismatch occurs with respect to a currently-viewed real-time image, regardless of the presence/absence of the forceps 13. Because the two images in which the difference therebetween has increased end up being combined in this case, the displayed image would be unnatural and hard to see.

Therefore, the endoscope apparatus 2 according to this embodiment is configured so that the most recent image can be provided, in which unnaturalness is removed by reducing the time difference between the saved image and the real-time image as much as possibly by successively updating the saved images. In the following, with regard to the endoscope apparatus 2 according to this embodiment, a description of commonalities with the endoscope apparatus 1 according to the first embodiment will be omitted, and the differences therefrom will mainly be described.

In the endoscope apparatus 2 according to this embodiment, as shown in Fig. 5, the control unit 30 is provided with, as its functions, a characteristic-marker-to-forceps-distance calculating portion (treatment-position detecting portion) 41, a saved-image-rewrite judging portion (movement-level calculating portion) 42, a display-image combining portion 43, and a saved-image combining portion 44, in addition to the configuration shown in Fig. 2 (the configuration of the first embodiment).

In the control unit 30 having the above-described configuration, an image generated by the image generating portion 31 is sent to the forceps detecting portion 33 and the image-saving memory portion 32, as with the first embodiment. The image-saving memory portion 32 saves the images sent thereto for a certain duration, for example, about 5 seconds, starting from several seconds before reaching the current time.

As with the first embodiment, the forceps detecting portion 33 judges, by means of color recognition or the like, whether or not the forceps 13 exist in an image on the basis of the image sent thereto. If it is judged that the forceps 13 do not exist in the image, the image generated by the image generating portion 31 is sent to the monitor 25 without modification so that a real-time image is displayed on the monitor 25, and new images also are saved sequentially in the image-saving memory portion 32.

On the other hand, if the forceps detecting portion 33 judges that the forceps 13 exist in the images, the forceps detecting portion 33 stops writing new images in the image-saving memory portion 32, and also outputs, from the forceps detecting portion 33 to the image-saving memory portion 32, an instruction for retaining an image immediately before the forceps 13 were recognized. The saved image retained by the image-saving memory portion 32 in this case is sent to the intra-image-characteristic-marker recognition portion 34 from the image-saving memory portion 32. In addition, the forceps detecting portion 33 sends the real-time image at that time to the intra-image-characteristic-marker recognition portion 34.

With regard to the real-time image and the saved image, as with the first embodiment, the intra-image-characteristic-marker recognition portion 34 identifies portions that serve as characteristic markers in the images by selecting points where the luminance is higher than the surroundings, points where the color is different, and so forth, in the respective images. The real-time image and the saved image for which the characteristic markers are identified in this way are sent to the image-position aligning portion 35 from the intra-image-characteristic-marker recognition portion 34. In addition, the intra-image-characteristic-marker recognition portion 34 also judges the presence/absence of the forceps 13 by means of color recognition in addition to the characteristic-marker information, and sends the positional information for the characteristic markers and the positional information for the forceps tip to the characteristic-marker-to-forceps-distance calculating portion 41.

As with the first embodiment, the image-position aligning portion 35 aligns the positions of the real-time image and the saved image on the basis of the information about the characteristic markers. The real-time image and the saved image whose positions have been aligned by the image-position aligning portion 35 are sent to the forceps-region extracting portion 36.

With regard to the real-time image, the forceps-region extracting portion 36 extracts the outline of the forceps 13 by utilizing a color difference, as with the forceps detecting portion 33. Specifically, the forceps-region extracting portion 36 extracts the outline of the forceps region (treatment-instrument region) by distinguishing the border between the forceps 13 and the biological-subject portion on the basis of the color difference between the forceps 13 and the biological subject.

In addition, the forceps-region extracting portion 36 extracts a portion corresponding to the forceps region in the saved image on the basis of the information about the extracted forceps region of the real-time image. This is preparation for subsequently cutting out the image region corresponding to the forceps 13 in the real-time image from the saved image.

On the other hand, on the basis of the positional information for the characteristic marker (× mark 51) and the positional information for the tip of the forceps 13, sent from the intra-image-characteristic-marker recognition portion 34, the distance between these two points is calculated at the characteristic-marker-to-forceps-distance calculating portion 41, as shown in Fig. 6(c). The distance information calculated in this way is sent to the saved-image-rewrite judging portion 42.

Here, Fig. 6(a) shows a real-time image before insertion of the forceps; Fig. 6(b) shows a real-time image after insertion of the forceps; Fig. 6(c) shows an image in which a saved image is combined with the forceps region in the real-time image; and the Figs. 6(d) and 6(e) show an image in which the forceps have been moved in the image in Fig. 6(c).

The saved-image-rewrite judging portion 42 judges how much the forceps 13 have been moved in the image with respect to the characteristic marker, and, depending on this amount of change, determines whether or not the currently-saved saved image should be updated. A reference value for judging whether to rewrite the saved image depending on the amount of change can be freely set. Specifically, updating may be performed if, for example, the distance between the forceps 13 and the characteristic marker (× mark 51) has changed by ten pixels or more relative to the distance in the initial saved image. This is for recognizing how much the forceps 13 have been moved since the forceps 13 were first recognized, and the forceps movement indicates that it is now possible to obtain information about a portion behind the forceps, which could not be obtained in the real-time image until then.

By updating the saved image by using the information other than the forceps region in the real-time image in this way, the saved image possesses the most recent information. In other words, by detecting the movement of the forceps 13 in the real-time image and by updating the saved images one after another on the basis of that information, the saved images are always updated with the most recent information obtained from the current real-time image.

In the case of the first image obtained when the forceps 13 were recognized, data that serve as the reference for the saved image are obtained here. In other words, because the movement distance is zero, the saved image is not rewritten. The data that serve as the reference are saved in the saved-image-rewrite judging portion 42 as the reference data for rewrite judgment. Specifically, the distance between the characteristic point and the tip of the forceps 13 is calculated on the basis of the reference data, and if the distance in newly-sent image data is 10 pixels or greater relative to this distance, processing for updating the saved image is performed. Because the reference data are obtained here, a judgment result that the saved image should not be rewritten is obtained, and this result is sent to the forceps-region image processing portion 37.

The forceps-region image processing portion 37 performs the image-cut-out operation on the basis of the information about the image and the forceps region sent thereto from the forceps-region extracting portion 36. With regard to the real-time image, the image inside the outline of the forceps 13 is cut out, thereby leaving only the biological-subject portions. The image of the forceps 13 cut out here is not used because it makes the image of the biological subject invisible, hiding the biological-subject information.

With regard to the saved image, because the position thereof has been aligned with that of the real-time image and the portion thereof corresponding to the forceps region in the real-time image has been extracted, that portion is cut out. Thus, the portion that was not visible in the real-time image because it is behind the forceps 13 is taken out from the saved image. The real-time image from which the forceps 13 have been removed and the saved image which is a cutout showing the region corresponding to the portion that was not visible because it is behind the forceps 13 are sent to the display-image combining portion 43.

The display-image combining portion 43 combines the two images sent thereto. In this way, by pasting the image having the biological-subject information that was not visible because it is behind the forceps 13, taken from the saved image, into the image in which the forceps 13 have been removed from the real-time image, a combined image in which the portion behind the forceps 13 is visible is created.
Then, by sending the combined image to the monitor 25, the combined image in which the biological-subject image is pasted inside the outline, which indicates the forceps region, is displayed on the screen.

Next, the case in which the image generated at the image generating portion 31 has been processed at the individual processing portions in the same way as has previously been described and new image information is sent to the saved-image-rewrite judging portion 42 will be described. With regard to that image in this case, how much distance there is between the characteristic marker and the tip of the forceps 13 is calculated by the characteristic-marker-to-forceps-distance calculating portion 41, and by comparing that distance with the reference data for the currently-retained saved image, it is judged whether or not the forceps 13 have been moved. Then, whether or not the saved image should be updated is determined depending on the result thereof (distance moved by the forceps 13).

The result calculated by the characteristic-marker-to-forceps-distance calculating portion 41 is compared by the saved-image-rewrite judging portion 42. If the distance moved by the forceps 13 does not reach the reference value, for example, 10 pixels, it is judged that there is no particular change in visible components, and the judgment result that the saved image should not to be rewritten is sent to the forceps-region image processing portion 37.

On the other hand, if the distance moved by the forceps 13 exceeds the reference value of 10 pixels, it is judged that the forceps 13 have been moved. Specifically, because this means that the biological-subject information that was not visible before because it is behind the forceps 13 has become visible, the instruction for updating the saved image is sent to the forceps-region image processing portion 37. In addition, because the saved image will be updated, the reference value is also updated to a newly calculated value. Then, it is used for comparison with the next image data sent thereto.

The forceps-region image processing portion 37 performs the image-cut-out operation on the basis of the information about the image and the forceps region sent thereto from the forceps-region extracting portion 36. With regard to the real-time image, the image inside the outline of the forceps 13 is cut out, thereby leaving only the biological-subject portions. The image of the forceps 13 removed here is not used because it makes the image of the biological subject invisible, hiding the biological-subject information.

With regard to the saved image, because the position thereof has been aligned with that of the real-time image and the portion thereof corresponding to the forceps region in the real-time image has been extracted, that portion is cut out. Thus, the portion that was not visible in the real-time image because it is behind the forceps 13 is taken out from the saved image. The real-time image from which the forceps 13 have been removed and the saved image which is a cutout showing the region corresponding to the portion that was not visible because it is behind the forceps 13 are sent to the display-image combining portion 43. In addition, because the saved image is updated in response to the result from the saved-image-rewrite judging portion 42, the combined image is also sent to the saved-image combining portion 44.

A combined image is created from the two images sent to the display-image combining portion 43 from the forceps-region image processing portion 37, as has previously been described, and by sending that combined image to the monitor 25, the combined image in which the biological-subject image is pasted inside the outline, which indicates the forceps region, is displayed on the screen.

In addition, the two images are also sent to the saved-image combining portion 44. By similarly combining these images, an image to be saved, in which regions other than the forceps region are shown by the most recent real-time image, is created. However, the outline of the forceps 13, such as that in the display image, is not displayed. The image created here serves as a new saved image that provides information for the portion behind the forceps 13 for the subsequent real-time images. The newly created saved image is sent to the image-saving memory portion 32, where the saved image that has been retained up to that point is updated to the new saved image that is newly created this time.

The operation of the endoscope apparatus 2 having the above-described configuration will be described in accordance with a flowchart shown in Fig. 7. Fig. 7 shows a flowchart indicating steps from finding a diseased portion by means of endoscope observation to performing biopsy, employing the endoscope apparatus 2 of this embodiment.

First, once observation is started using the endoscope apparatus 2 of this embodiment, the illumination light from the laser light source 20 is radiated onto the subject A, and the reflected light from the subject A is detected by the color CCD 27 to be converted to image data. An image is generated at the image generating portion 31 on the basis of the image data, and the generated image of the subject A is displayed on the monitor 25 (Step S11).

The image is displayed on the monitor 25 as shown in Fig. 6(a), and a surgeon observes this image, searching for a biopsy target portion. At this time, images up to, for example, 5 seconds before the time of observation, are constantly saved in the image-saving memory portion 32 (Step S12).

In this state, the forceps detecting portion 33 judges whether or not the forceps 13 exist in an image (Step S13).
If the forceps 13 are not present in the image in Step S13, the real-time image is displayed on the monitor 25 without modification (Step S25).

In the case in which the biopsy target site is found within the observation viewing field in the real-time image, the forceps 13 are inserted into the forceps inlet 14 in the endoscope 10 so that the forceps 13 are inserted through to the tip of the inserted portion 11. By doing so, the forceps 13 appear within the observation viewing field, as shown in Fig. 6(b). The forceps detecting portion 33 recognizes the presence/absence of the forceps 13 in the observation viewing field by means of a color change or the like.

In the case in which the forceps 13 are present in the image in Step S13, the saved image immediately before the appearance of the forceps 13 is read out from the image-saving memory portion 32 (Step S14).
Next, the intra-image-characteristic-marker recognition portion 34 sets the characteristic markers in the read-out saved image and the real-time image by utilizing points where the luminance is higher than the surroundings, points where the color is different, and so forth (Step S15).

Next, the characteristic-marker-to-forceps-distance calculating portion 41 calculates the distance between the characteristic marker and the forceps 13 for each of the saved image and the real-time image, as shown in Fig. 6(c) (Step S16).
Next, on the basis of these calculation results, the saved-image-rewrite judging portion 42 judges, for each of the saved image and the real-time image, whether or not the distance between the characteristic marker and the forceps 13 is equal to or greater than the reference value (for example, 10 pixels) (Step S17).

In Step S17, if the distance between the characteristic marker and the forceps 13 is less than the reference value, a combined image is created in the same way as the previously performed processing (Step S24).
On the other hand, if the distance between the characteristic marker and the forceps 13 is equal to or greater than the reference value, the instruction for updating the saved image is issued, and the image-position aligning portion 35 aligns the positions of the saved image and the real-time image on the basis of the set characteristic markers (Step S18).

Next, the forceps-region extracting portion 36 extracts the outline of the forceps 13 and the forceps region from the real-time image on the basis of the color difference between the forceps 13 and the biological subject (Step S19). This forceps region is also used when performing the cut-out operation in the saved image.

Next, the forceps region is cut out from the real-time image, thereby leaving the remaining biological-subject portions (Step S20).
Next, the portion corresponding to the forceps region is cut out from the saved image, and that cut-out biological-subject information is pasted into the image having the remaining biological-subject information of the real-time image (Step S21). The image combined in this way is saved in the image-saving memory portion 32 as a new saved image.
Next, the border portion where the two images are combined is displayed on the screen of the monitor 25 as the outline of the forceps 13 (Step S22).

Next, image display is switched from the real-time display to an image-overlaying mode (Step S23). When the display is switched to the image-overlaying mode, the forceps 13 are switched to the outline display, which makes the portion hidden by the forceps 13 visible, as shown in Fig. 6(e). By doing so, the surgeon can advance the forceps 13 toward the biopsy target site and perform biopsy, while viewing the screen in which the portion that was hidden by the forceps 13 has become visible.

Note that, because the forceps 13 disappear from the screen when the biopsy is completed, the absence of the forceps 13 is recognized, and the screen display is switched from the image-overlaying mode to the real-time display.

As described above, the features of the endoscope apparatus 2 according to this embodiment include not only that display thereof is such that the positional relationship between the biopsy target site and the forceps 13 can be visually recognized, even in cases such as when the biopsy target site is not visible due to the forceps 13, but also that the saved image is updated by making a judgment therefor on the basis of the movement level of the forceps 13. By doing so, in the case in which, the movement of the forceps 13 makes it possible to acquire an image of a location that has been hidden up to that point, a new image of the location that has been hidden can be acquired, and that image can be saved in the image-saving memory portion 32. Accordingly, the saved images saved in the image-saving memory portion 32 can be constantly updated, and, by combining images by using them by means of the display-image combining portion 43, the biological-subject information of the location hidden by the forceps 13 can be displayed by using images having as little time difference as possible. Accordingly, it is possible to obtain natural images in accordance with changes in the influence of the light distribution, changes in the diseased portion, and so forth, which makes it possible to enhance observation precision.

Note that, with the endoscope apparatus 2 according to this embodiment, the image-saving memory portion 32 may update the images to be saved at predetermined intervals.
By doing so, the saved image used for combining by the display-image combining portion 43 can be updated at the predetermined intervals, which makes it possible to display the biological-subject information of the location hidden by the forceps 13 by using the images having as little time difference as possible. In addition, because it is not necessary to detect the position of the forceps 13, the amount of processing for the apparatus as a whole can be reduced, which enables smooth endoscope observation.

### {Third Embodiment}

Next, an endoscope apparatus 3 according to a third embodiment of the present invention will be described by using Figs. 8 to 12.
Fig. 8 is a functional block diagram of the endoscope apparatus 3 according to the embodiment, and the configuration thereof is the same as that of the endoscope apparatus 1 according to the first embodiment, except for the processing in the control unit 30.

The endoscope apparatus 3 according to the third embodiment is the same as the endoscope apparatus 1 according to the first embodiment described above in terms of the processing up to the generation of the observation image.
Here, with the first embodiment, when the forceps 13 exit the tip of the endoscope 10, the image is affected because the distribution of the illumination light is affected. Specifically, because the forceps 13 block the illumination light or cause scattering thereof with respect to an observation portion, which creates a portion made darker due to shading by the forceps 13 and a portion made brighter due to strong illumination by the scattered light caused by the forceps 13. Because of this, the manner in which an observation subject appears differs between when the forceps 13 are present and when they are absent. In addition, a region that becomes saturated by becoming excessively bright due to the presence of the forceps 13 also occurs. With regard to this region, information about the form thereof ends up being lost. Therefore, in this embodiment, the influence of blocking of the illumination light and that of scattered light due to the appearance of the forceps 13 is reduced.

First, the situation involved here can be roughly divided into two cases. In the first case, a region where the brightness of the observation portion is changed due to the appearance of the forceps 13 (becoming brighter or darker as compared with when the forceps 13 were absent) occurs. In the second case, the information about form is lost due to saturation caused by the appearance of the forceps 13 (becoming excessively bright due to the influence of the forceps 13). In the following, with regard to the endoscope apparatus 3 according to this embodiment, a description of commonalities with the endoscope apparatus 1 according to the first embodiment will be omitted, and the differences therefrom will mainly be described.

In the endoscope apparatus 3 according to this embodiment, as shown in Fig. 8, the control unit 30 is provided with, as its functions, a region-dividing portion 51, a region-wise histogram generating portion (gradation-value analyzing portion, gradation-value detecting portion) 52, a corresponding-region-wise histogram comparing portion 53, a corresponding-region-wise adjusting portion (gradation-value adjusting portion) 54, a forceps-and-saturation-region extracting portion 55, and a forceps-and-saturation-region image processing portion 56, in addition to the configuration shown in Fig. 2 (the configuration of the first embodiment).

In the control unit 30 having the above-described configuration, an image generated by the image generating portion 31 is sent to the forceps detecting portion 33 and the image-saving memory portion 32, as with the first embodiment. The image-saving memory portion 32 saves the images sent thereto for a certain duration, for example, about 5 seconds, starting from several seconds before reaching the current time.

As with the first embodiment, the forceps detecting portion 33 judges, by means of color recognition or the like, whether or not the forceps 13 exist in an image on the basis of the image sent thereto. If it is judged that the forceps 13 do not exist in the image, the image generated by the image generating portion 31 is sent to the monitor 25 without modification so that a real-time image is displayed on the monitor 25, and new images also are saved sequentially in the image-saving memory portion 32.

On the other hand, if the forceps detecting portion 33 judges that the forceps 13 exist in the images, the forceps detecting portion 33 stops writing new images in the image-saving memory portion 32 and also outputs, from the forceps detecting portion 33 to the image-saving memory portion 32, an instruction for retaining an image immediately before the forceps 13 were recognized. The saved image retained by the image-saving memory portion 32 in this case is sent to the intra-image-characteristic-marker recognition portion 34 from the image-saving memory portion 32. In addition, the forceps detecting portion 33 sends the real-time image at that time to the intra-image-characteristic-marker recognition portion 34.

With regard to the real-time image and the saved image, as with the first embodiment, the intra-image-characteristic-marker recognition portion 34 identifies portions that serve as characteristic markers in the images by selecting points where the luminance is higher than the surroundings, points where the color is different, and so forth, in the respective images. The real-time image and the saved image for which the characteristic markers are identified in this way are sent to the image-position aligning portion 35 from the intra-image-characteristic-marker recognition portion 34.

As with the first embodiment, the image-position aligning portion 35 aligns the positions of the real-time image and the saved image on the basis of the information about the characteristic markers. The real-time image and the saved image whose positions have been aligned by the image-position aligning portion 35 are sent to the region-dividing portion 51.

The region-dividing portion 51 divides both the saved image and the real-time image sent thereto into multiple regions, as shown in Figs. 9 and 10. An appropriate value is set as the number of divisions in consideration of the resolution, and the same region-dividing processing is applied to the saved image and the real-time image. Because the positions of the saved image and the real-time image are aligned in advance, each of the divided regions therein is correspondingly positioned. The saved image and the real-time image that have been divided into the multiple regions are sent to the region-wise histogram generating portion 52.

Here, Fig. 9 shows a state in which the saved image is divided into the multiple regions, and Fig. 10 shows a state in which the real-time image is divided into multiple regions. In Figs. 9 and 10, reference sign B indicates a diseased portion, reference sign 61 indicates a region made darker due to shading by the forceps 13, reference sign 62 indicates a region made brighter due to reflection caused by the forceps 13, and the reference sign 63 indicates a saturated region (saturation region).

The region-wise histogram generating portion 52 creates histograms of gradation values for the individual regions in the saved image and the real-time image sent thereto, such as those shown in Fig. 11. In Fig. 11, reference sign 58 indicates a histogram of gradation values for the real-time image, and reference sign 59 indicates a histogram of gradation values for the saved image. The histograms of gradation values created in this way are sent to the corresponding-region-wise histogram comparing portion 53.

With regard to the saved image and the real-time image, the corresponding-region-wise histogram comparing portion 53 compares histograms for each of corresponding regions. As shown in Fig. 11, in the case in which the histogram of the real-time image is shown to be shifted with respect to that of the saved image, when the corresponding regions are compared between the saved image and the real-time image, the real-time image is affected by the presence of the forceps 13, thus being a brighter (or darker) image. Therefore, the corresponding-region-wise histogram comparing portion 53 calculates the shift therebetween. Here, the histogram-shift level between the saved image and the real-time image calculated here is sent to the corresponding-region-wise adjusting portion 54.

In addition, the saturated regions (saturation regions) where the gradation values have become saturated can also be identified by comparing the histograms. Therefore, the corresponding-region-wise histogram comparing portion 53 also extracts regions where the information about the form thereof has been lost due to saturation and sends that region information to the forceps-and-saturation-region extracting portion 55.

The corresponding-region-wise adjusting portion 54 adjusts the histograms for the real-time image and the saved image on the basis of the image information and the histogram-shift levels sent thereto. For example, as shown in Fig. 11, the brightness is increased in regions in the real-time image that are excessively dark so as to be approximately equal to corresponding regions in the saved image. Similarly, the brightness is decreased in regions in the real-time image that are excessively bright so as to be approximately equal to corresponding regions in the saved image. The images created here are sent to the forceps-and-saturation-region extracting portion 55.

In the real-time image, the forceps-and-saturation-region extracting portion 55 extracts the outline of the forceps 13 by utilizing a color difference, as with the forceps detecting portion 33. Specifically, the outline of the forceps region is extracted by distinguishing the border between the forceps 13 and the biological-subject portion on the basis of the color difference between the forceps 13 and the biological subject. Because the region of the forceps 13 is extracted in this way from the real-time image, on the basis of that information, a portion corresponding to the forceps region is extracted from the saved image in which histogram adjustment has been performed. This is preparation for subsequently cutting out the image region corresponding to the forceps region in the real-time image from the saved image, for which histogram adjustment has been performed.

In addition, the forceps-and-saturation-region extracting portion 55 extracts regions in which saturation has occurred in the real-time image by using the saturation-region information detected by the corresponding-region-wise histogram comparing portion 53. That image to which the information about the forceps region and the saturation region has been added is sent to the forceps-and-saturation-region image processing portion 56.

On the basis of the images and the forceps-region information sent thereto from the forceps-and-saturation-region extracting portion 55, the forceps-and-saturation-region image processing portion 56 performs image processing in which the image inside the outline of the forceps 13 is cut out, thereby leaving only the biological-subject portions. Then, image processing is performed on the basis of the saturation-region information, in which portions for which the information about the form thereof has been lost are cut out, thereby leaving portions that are biological-subject portions and that also retain the information about the form thereof where saturation is not occurring. The images processed here are sent to the image combining portion 38.

The image combining portion 38 combines the real-time image and the saved image by using the image information sent thereto from the forceps-and-saturation-region image processing portion 56. By pasting the biological-subject information for the portion behind the forceps, which has been taken out from the saved image in which histogram adjustment has been performed, into the portion where the forceps 13 have been removed from the real-time image, a combined image in which the portion behind the forceps 13 is visible is created. In addition, by also performing image combining for portions having the saturation regions, in which corresponding portions are pasted by using the saved image immediately before the forceps 13 were recognized, it is possible to supplement the portions in the real-time image for which the information about the form thereof has been lost due to saturation caused by the appearance of the forceps 13.

By sending the image combined in this way to the monitor 25, the combined image in which the biological-subject image is pasted inside the outline, which indicates the forceps region, and in which adjustment for suppressing the influence of a brightness change caused by the forceps 13 has been performed is displayed on the screen.

The operation of the endoscope apparatus 3 having the above-described configuration will be described in accordance with a flowchart shown in Fig. 12. Fig. 12 shows a flowchart indicating steps from finding a diseased portion by means of endoscope observation to performing biopsy, employing the endoscope apparatus 3 of this embodiment. With the third embodiment, the same processing as that in the first and the second embodiments is executed until it is judged that the forceps 13 are present on the screen.

First, once observation is started using the endoscope apparatus 3 of this embodiment, the illumination light from the laser-light source 20 is radiated onto the subject A, and the reflected light from the subject A is detected by the color CCD 27 to be converted to image data. An image is generated at the image generating portion 31 on the basis of the image data, and the generated image of the subject A is displayed on the monitor 25 (Step S31).

The image is displayed on the monitor 25 as shown in Fig. 3(a), and a surgeon observes this image, searching for a biopsy target site. At this time, images up to, for example, 5 seconds before the time of observation, are constantly saved in the image-saving memory portion 32 (Step S32).

In this state, the forceps detecting portion 33 judges whether or not the forceps 13 exist in an image (Step S33).
If the forceps 13 are not present in the image in Step S33, the real-time image is displayed on the monitor 25 (Step S45).

In the case in which the biopsy target site is found within the observation viewing field in the real-time image, the forceps 13 is inserted into the forceps inlet 14 in the endoscope 10 so that the forceps 13 is inserted through to the tip of the inserted portion 11. By doing so, the forceps 13 appears within the observation viewing field, as shown in Fig. 3(b). The forceps detecting portion 33 recognizes the presence/absence of the forceps 13 in the observation viewing field by means of a color change in the image or the like.

In the case in which the forceps 13 are present in the image in Step S33, the saved image immediately before the appearance of the forceps 13 is read out from the image-saving memory portion 32 (Step S34).
Next, the intra-image-characteristic-marker recognition portion 34 sets the characteristic markers in the read-out saved image and the real-time image by utilizing points where the luminance is higher than the surroundings, points where the color is different, and so forth (Step S35). In addition, the positions of the saved image and the real-time image are aligned by the image-position aligning portion 35 on the basis of the set characteristic markers.

Next, the saved image and the real-time image are divided into similar regions, histograms for the respective regions are created, and these histograms are compared (Step S36). By comparing histograms for the corresponding regions, it is possible to distinguish whether the real-time image is brighter or darker than the saved image or whether or not saturation has occurred, causing a loss of the information about the form.

The regions in the real-time image that have been judged to be saturated regions in Step S36 are switched with images of corresponding regions in the saved image (Step S42). Then, as with the first embodiment, cut-out and pasting of the forceps region are performed, in which the corresponding portions are cut out from the saved image and pasted into the forceps region of the real-time image (Step S43).

With regard to the regions in the real-time image that have been judged not to be saturated regions in Step S36, histogram adjustment for the image is performed for the individual corresponding regions (Step S37). Specifically, histograms of the regions for which the results of histogram comparisons indicate that the regions are darker or brighter than those of the saved image are adjusted so that the brightness thereof becomes equivalent to that of the saved image.

Next, in the real-time image whose histograms have been adjusted, the outline of the forceps 13 is extracted, and the region of the forceps 13 is also extracted, on the basis of the color difference between the forceps 13 and the biological subject (Step S38). These regions are also used when performing the cut-out operation in the saved image.

Next, the forceps region is removed from the real-time image whose histograms have been adjusted, thereby leaving the remaining biological-subject portions (Step S39).
Then, the portion corresponding to the forceps region is cut out from the saved image, and the cut-out biological-subject information is pasted into the image having the remaining biological-subject information of the real-time image (Step S40). In addition, because the boundary portion of the two combined images forms the outline of the forceps 13, the outline display is also performed on the screen of the monitor 25.

Next, image display is switched from the real-time display to an image-overlaying mode (Step S41). When the display is switched to the image-overlaying mode, the forceps 13 are switched to the outline display, which makes the portion hidden by the forceps 13 visible, as shown in Fig. 3(e). By doing so, the surgeon can advance the forceps 13 toward the biopsy target site and perform biopsy, while viewing the screen in which the portion that was hidden by the forceps 13 has become visible.

Note that, because the forceps 13 disappear from the screen when the biopsy is completed, the absence of the forceps 13 is recognized, and the screen display is switched from the image-overlaying mode to the real-time display.

As described above, with the endoscope apparatus 3 according to this embodiment, the histograms of gradation values in the real-time image and the histograms of gradation values in the saved image saved in the image-saving memory portion 32 can be made similar. By doing so, it is possible to correct gradation values of regions made darker by the shadow of the forceps 13, as well as those of regions made brighter by reflected light from the forceps. Accordingly, changes in illumination conditions due to the positions of the forceps 13 are suppressed, which makes it possible to display an image under uniform conditions. In other words, it is possible to reduce the differences in the way the image appears due to the influence on the distribution of the illumination light caused by the presence of the forceps 13, as well as the influence of scattering of the illumination light caused by the forceps 13, and it is possible to provide an image that is not unnatural and that is easy to view.

In addition, in the case in which saturated regions exist in a real-time image, where the gradation values are saturated, images of the saturated regions can be replaced with saved images by means of the image combining portion 38. By doing so, even for the regions for which information about the form thereof has been lost due to the saturation, because the information about the form can be acquired by switching the images, the state of a biological subject can be ascertained regardless of the illumination conditions.
Note that although replacement in the real-time image is performed by using the saved image in this embodiment, all divided regions may be replaced by using a representative value, for example, an average value or the like, obtained from the saved histograms obtained for each of the divided regions.

### {Fourth Embodiment}

Next, an endoscope apparatus 4 according to a fourth embodiment of the present invention will be described by using Figs. 13 to 16.
Fig. 13 is a functional block diagram of the endoscope apparatus 4 according to the embodiment, and the configuration thereof is the same as that of the endoscope apparatus 1 according to the first embodiment, except for the processing in the control unit 30.

The endoscope apparatus 4 according to this embodiment is the same as the endoscope apparatus 1 according to the first embodiment described above in terms of the processing up to the generation of the observation image.
Here, with the first to third embodiments, if the tip of the endoscope 10 is moved, because the saved image does not include a corresponding area, it is not possible to display the portion behind the forceps 13. For example, although the saved image is updated in the second embodiment, this image can be used in the case in which viewing fields are the same for the real-time image and the saved image and they can be compared. However, if the tip of the forceps 13 is moved considerably, because the forceps 13 exist in a portion other than the region in the saved image, it is not possible to provide biological subject information to be pasted into the portion of the forceps 13, which creates a problem in that the portion behind the forceps cannot be displayed.

Therefore, this embodiment is configured such that images are saved from the beginning of the observation; an image having a wider angle of view, which includes the area of the viewing field with which real-time observation is being performed, is searched for among them; an image is taken out from that image and is enlarged so as to correspond to the real-time image, after which the biological subject information thereof is pasted into the forceps region. By doing so, even with an image that cannot be dealt with using several-seconds worth of saved images when the tip of the endoscope 10 is moved considerably, it is possible to handle such an image, and an image showing the portion behind the forceps can be combined.

In the endoscope apparatus 4 according to this embodiment, as shown in Fig. 13, the control unit 30 is provided with, as its functions, an intra-image-characteristic-marker seeking portion (characteristic-point search portion) 65, a enlargement ratio setting portion 66, and an image enlarging portion 67, in addition to the configuration shown in Fig. 2 (the configuration of the first embodiment).

In the control unit 30 having the above-described configuration, an image generated by the image generating portion 31 is sent to the forceps detecting portion 33 and the image-saving memory portion 32, as with the first embodiment. The image-saving memory portion 32 saves all images that have been created since the beginning of the examination.

As with the first embodiment, the forceps detecting portion 33 judges, by means of color recognition or the like, whether or not the forceps 13 exist in an image on the basis of the image sent thereto. If it is judged that the forceps 13 do not exist in the image, the image generated by the image generating portion 31 is sent to the monitor 25 without modification so that a real-time image is displayed on the monitor 25, and new images also are saved sequentially in the image-saving memory portion 32.

On the other hand, if the forceps detecting portion 33 judges that the forceps 13 exist in the images, the forceps detecting portion 33 stops writing new images in the image-saving memory portion 32, and also outputs, from the forceps detecting portion 33 to the image-saving memory portion 32, an instruction for retaining an image immediately before the forceps 13 were recognized. The saved image retained by the image-saving memory portion 32 in this case is sent to the intra-image-characteristic-marker recognition portion 34 from the image-saving memory portion 32. In addition, the forceps detecting portion 33 sends the real-time image of that time to the intra-image-characteristic-marker recognition portion 34.

With regard to the real-time image and the saved image, the intra-image-characteristic-marker recognition portion 34 identifies two characteristic markers each in the respective images by selecting points where the luminance is higher than the surroundings, points where the color is different, and so forth. For example, in the example shown in Fig. 14, an example real-time image corresponds to Fig. 14(c) and an example saved image corresponds to Fig. 14(a). In addition, circular marks 71 and triangular marks 72 in the figures indicate marker portions that serve as the characteristic points. The real-time image and the saved image, in which two characteristic points are identified in the same image in this way, are sent to the intra-image-characteristic-marker seeking portion 65.

As shown in Fig. 14(c), the intra-image-characteristic-marker seeking portion 65 determines distances between the two markers in the images on the basis of the characteristic markers. By comparing the distance calculated in the real-time image and the distance calculated in the saved image, the enlargement ratio by which the saved image is enlarged relative to the real-time image can be determined.

In addition, as shown in Fig. 14(d), the intra-image-characteristic-marker seeking portion 65 calculates angles and distances between characteristic markers of the real-time image and the four corners of the image. Then, it is confirmed whether or not the saved image includes the region of the real-time image when the angles and the distances with respect to the four corners are corrected for the saved image using the previously determined enlargement ratio. If the results match between the real-time image and the saved image (matching here means that there is no shifting of the viewing field due to the movement of the endoscope tip), because it can be judged that the currently retained saved image possesses the information about the portion behind the forceps for the real-time image, the same processing as that in the above-described first embodiment will be performed hereafter.

On the other hand, if the distances and angles between the four corners do not match between the real-time image and the saved image, because an appropriate saved image needs to be newly searched for from the image-saving memory portion 32, the intra-image-characteristic-marker seeking portion 65 searches for an image including the region of the real-time image from the image-saving memory portion 32.

The intra-image-characteristic-marker seeking portion 65 searches for an image including the characteristic markers from the image-saving memory portion 32 in such a manner as to track back in time. Then, if the characteristic markers are found, as has previously been described, the distances between the characteristic markers are measured, and the enlargement ratio by which the saved image is enlarged relative to the real-time image is calculated. Next, it is judged whether or not the saved image is an image including the region of the real-time image when the saved image is set to the determined enlargement ratio, on the basis of the information about the distances and angles between the characteristic points, determined in advance, in the real-time image and the four corners thereof.

If a found saved image does not include the real-time image, the intra-image-characteristic-marker seeking portion 65 continues to search for other saved images, and if the region of the real-time image is included, the intra-image-characteristic-marker seeking portion 65 sends the saved image obtained by the search to the enlargement-ratio setting portion 66.

The enlargement-ratio setting portion 66 sets the enlargement ratio determined by comparing the distances between the characteristic points in the real-time image and the distances between the characteristic points in the saved image.
As shown in Fig. 14(e), the image enlarging portion 67 enlarges the saved image obtained by the search on the basis of the enlargement ratio set by the enlargement-ratio setting portion 66. The enlarged image is sent to the image-position aligning portion 35.
Because the subsequent image processing is that same as that in the first embodiment described above, the description thereof will be omitted.

By sending an image combined in this way to the monitor 25, a combined image in which the biological-subject image is pasted inside the outline, which indicates the forceps region, is displayed. In addition, because the combined image can be created from the most appropriate saved image on the basis of the currently detected characteristic markers from the past saved images, a display in which the positional relationship between the forceps 13 and the target site can be observed is possible, even if the tip of the endoscope 10 is moved.

The operation of the endoscope apparatus 4 having the above-described configuration will be described in accordance with flowcharts shown in Figs. 15 and 16. Figs. 15 and 16 show flowcharts indicating steps from finding a diseased portion by means of endoscope observation to performing biopsy, employing the endoscope apparatus 4 of this embodiment. With the fourth embodiment, the same processing as that in the first to the third embodiments is executed until it is judged that the forceps 13 are present in the image.

First, once observation is started using the endoscope apparatus 1 of this embodiment, the illumination light from the laser light source 20 is radiated onto the subject A, and the reflected light from the subject A is detected by the color CCD 27 to be converted to image data. An image is generated at the image generating portion 31 on the basis of the image data, and the generated image of the subject A is displayed on the monitor 25 (Step S51).

The image is displayed on the monitor 25 as shown in Fig. 3(a), and a surgeon observes this image, searching for a biopsy target site. At this time, all images up to the present are saved from the beginning of the observation (Step S52).

In this state, the forceps detecting portion 33 judges whether or not the forceps 13 exist in an image (Step S53).
If the forceps 13 are not present in the image in Step S53, the real-time image is displayed on the monitor 25 (Step S66).

In the case in which the biopsy target site is found within the observation viewing field in the real-time image, the forceps 13 are inserted into the forceps inlet 14 in the endoscope 10 so that the forceps 13 are inserted through to the tip of the inserted portion 11. By doing so, the forceps 13 appear within the observation viewing field, as shown in Fig. 3(b). The forceps detecting portion 33 recognizes the presence/absence of the forceps 13 in the observation viewing field by means of a color change or the like.

In the case in which the forceps 13 are present in the image in Step S53, the saved image immediately before the appearance of the forceps 13 is read out from the image-saving memory portion 32 (Step S54).
Next, the intra-image-characteristic-marker recognition portion 34 sets two characteristic markers in the read-out saved image and the real-time image by utilizing points where the luminance is higher than the surroundings, points where the color is different, and so forth (Step S55).

Next, it is judged whether the saved image corresponds to the real-time image (Step S56). Specifically, the distances between the two characteristic markers are compared between the real-time image and the saved image, and the enlargement ratio of the saved image relative to the real-time image is calculated. Then, the distances and angles from the characteristic markers in the real-time image to the four corners of the image are calculated, and it is judged whether or not the area thereof is included in the saved image, taking the enlargement ratio into consideration.

If the saved image corresponds to the real-time image in Step S56, that is, if the above-described area is included in the saved image, because that indicates correspondence to the real-time image, images are processed, combined, and displayed, in the same way as in the first embodiment (Step S65).

On the other hand, if the saved image does not correspond to the real-time image in Step S56, that is, if all regions of the real-time image are not included in the saved image (if the viewing field of the real-time image falls, even slightly, outside the saved image), it is assumed that the currently retained saved image does not achieve correspondence, and an appropriate image is searched for from the image-saving memory portion 32, which continuously saves images from the start of the observation (Step S57).

A saved image including the two characteristic markers is searched for also when searching the image-saving memory portion 32, in the same way as has previously been done, and the enlargement ratio is calculated for that image by calculating the distance between the two points (Step S58). Then, it is judged whether or not the region of the real-time image is included in the image enlarged by that enlargement ratio.

Next, because the enlargement ratio is determined for the searched saved image, the searched saved image is enlarged by that enlargement ratio (Step S59).
Next, the positions of the enlarged saved image and the real-time image are aligned on the basis of the characteristic markers (Step S60).

Next, the outline of the forceps 13 is extracted in the real-time image on the basis of the color difference between the forceps 13 and the biological subject, and the region of the forceps 13 is also extracted (Step S61). This region is also used when performing the cut-out operation in the enlarged saved image.

Next, the portion corresponding to the forceps region is cut out from the enlarged saved image (Step S62).
Then, the forceps region is cut out from the real-time image, thereby leaving the remaining biological-subject portions, and the biological-subject information cut out from the saved image is pasted into the image having the remaining biological subject information of the real-time image (Step S63). In addition, because the boundary portion where the two images are combined forms the outline of the forceps 13, the outline display is also performed on the screen of the display monitor 25.

Next, image display is switched from the real-time display to an image-overlaying mode (Step S64). When the display is switched to the image-overlaying mode, the forceps 13 are switched to the outline display, which makes the portion hidden by the forceps 13 visible, as shown in Fig. 3(e). By doing so, the surgeon can advance the forceps 13 toward the biopsy target site and perform biopsy, while viewing the screen in which the portion that was hidden by the forceps 13 has become visible.

Note that, because the forceps 13 disappear from the screen when the biopsy is completed, the absence of the forceps 13 is recognized, and the screen display is switched from the image-overlaying mode to the real-time display.

As described above, with the endoscope apparatus 4 according to this embodiment, even in the case in which the characteristic points of the real-time image are not found in the most recent saved image saved in the image-saving memory portion 32, a saved image having the characteristic points is searched for among the plurality of saved saved images, and the forceps region can be extracted from that saved image and combined with the real-time image. Accordingly, the biological-subject information of the forceps region can be displayed even in the case in which the images have considerably changed due to considerable movement of the tip of the endoscope 10 or the case in which the angle of view has changed.

In addition, by enlarging or reducing the saved image by means of the image enlarging portion 67 and by extracting a region corresponding to the forceps region from the enlarged or reduced saved image, the region corresponding to the forceps region can be extracted from the enlarged or reduced saved image and combined with the real-time image, even in the case in which the size of the saved image and the size of the real-time image are different.

Although the individual embodiments of the present invention have been described above in detail with reference to the drawings, the specific configuration thereof is not limited to these embodiments, and design alterations or the like within a range that does not depart from the spirit of the present invention are also encompassed.
For example, although the outline of the forceps 13 is displayed in the individual embodiments in order to display the information about biological-subject portion hidden behind the forceps, any display method may be employed so long as the display is such that information about the biological-subject portion hidden behind the forceps 13 is made visible; for example, the forceps 13 may be semi-transparently displayed and overlaid with an image of the biological subject portion.

By doing so, a user can visually ascertain the biological subject portion behind the forceps 13 and the position of the forceps 13 can also be semi-transparently displayed in a combined image. Accordingly, three-dimensional information, such as the shape of the forceps 13 and so forth, can also be displayed, which makes it easier to ascertain the position and orientation of the forceps 13, and thus, biopsy can be performed more accurately.

In addition, although the individual embodiments have been described assuming that an image in which the forceps region is removed from the real-time image and an image which is the region corresponding to the forceps cut out from a saved image are combined by means of the image combining portion 38 or the display-image combining portion 43, the image which is the region corresponding to the forceps 13 cut out from a saved image may be combined with a real-time image from which the forceps region has not been removed.

In addition, although the individual embodiments have been described assuming that biopsy forceps are employed as a treatment instrument, it is not limited thereto, and any treatment instrument may be employed so long as it blocks the viewing field during endoscope observation. For example, because a portion behind a treatment instrument can also be displayed when using grasping forceps, a knife, a clip, a tube, a basket, a snare, and so forth, in addition to the biopsy forceps, the treatment accuracy can be enhanced.

### {Reference Signs List}

1, 2, 3, 4 endoscope apparatus
10 endoscope
20 light-source device
24 image-capturing optical system
25 monitor
27 color CCD
30 control unit
31 image generating portion (image acquisition portion)
32 image-saving memory portion (image saving portion)
33 forceps-detecting portion (treatment-instrument detecting portion)
34 intra-image-characteristic-marker recognition portion (characteristic-point detecting portion)
35 image-position aligning portion
36 forceps-region extracting portion (treatment-instrument-region extracting portion, treatment-instrument-corresponding-region extracting portion)
37 forceps-region image processing portion (image processing portion)
38 image combining portion
41 characteristic-marker-to-forceps-distance calculating portion (treatment-instrument-position detecting portion)
42 saved-image-rewrite judging portion (movement-level calculating portion)
43 display-image combining portion
44 saved-image combining portion
51 region-dividing portion
52 region-wise histogram generating portion (gradation-value analyzing portion, gradation-value detecting portion)
53 corresponding-region-wise histogram comparing portion
54 corresponding-region-wise adjusting portion (gradation-value adjusting portion)
55 forceps-and-saturation-region extracting portion
56 forceps-and-saturation-region image processing portion 65 intra-image-characteristic-marker seeking portion (characteristic-point search portion)
66 enlargement-ratio setting portion
67 image enlarging portion
A subject
B diseased portion

## Claims

1. An endoscope apparatus comprising:
an image acquisition portion that acquires an image of an subject;
an image saving portion that saves a current image acquired by the image acquisition portion;
a treatment-instrument-region extracting portion that extracts a treatment-instrument region in which a treatment instrument exists from the current image acquired by the image acquisition portion;
an image-position aligning portion that aligns positions of the saved image saved in the image saving portion and the current image acquired by the image acquisition portion;
a treatment-instrument-corresponding-region extracting portion that extracts a region corresponding to the treatment-instrument region from the saved image saved in the image saving portion; and
an image combining portion that combines an image of the region extracted by the treatment-instrument-corresponding-region extracting portion and the current image acquired by the image acquisition portion.

2. An endoscope apparatus according to Claim 1, further comprising:
an image processing portion that generates an image in which the treatment-instrument region extracted by the treatment-instrument-region extracting portion is removed from the current image acquired by the image acquisition portion,
wherein the image combining portion combines the image of the region extracted by the treatment-instrument-corresponding-region extracting portion and the image generated by the image processing portion.

3. An endoscope apparatus according to Claim 1, wherein
the image combining portion overlays positional information of the treatment-instrument region on the combined image.

4. An endoscope apparatus according to Claim 3, wherein
the image combining portion overlays an outline of the treatment instrument as the positional information of the treatment-instrument region.

5. An endoscope apparatus according to Claim 3, wherein
the image combining portion semi-transparently overlays the treatment instrument as the positional information of the treatment-instrument region.

6. An endoscope apparatus according to Claim 1, further comprising
a characteristic-point detecting portion that detects characteristic points in the current image and the saved image, wherein
the image-position aligning portion aligns the positions of the current image and the saved image by using the characteristic points detected by the characteristic-point detecting portion.

7. An endoscope apparatus according to Claim 1, further comprising:
a treatment-instrument detecting portion that detects the presence/absence of the treatment instrument in the current image, wherein
in the case in which the treatment-instrument detecting portion detects the treatment instrument, the treatment-instrument-region extracting portion extracts the treatment-instrument region from the current image.

8. An endoscope apparatus according to Claim 7, wherein
the treatment-instrument detecting portion detects the treatment instrument on the basis of color information of the current image.

9. An endoscope apparatus according to Claim 1, further comprising:
a treatment-instrument-position detecting portion that detects the position of the treatment instrument in the current image; and
a movement-level calculating portion that calculates the movement level of the treatment instrument on the basis of the position of the treatment instrument detected by the treatment-instrument-position detecting portion, wherein
in the case in which the movement level calculated by the movement-level calculating portion is equal to or greater than a predetermined distance, the image saving portion updates an image to be saved.

10. An endoscope apparatus according to Claim 1, wherein
the image saving portion updates an image to be saved at predetermined intervals.

11. An endoscope apparatus according to Claim 1, further comprising:
a region-dividing portion that divides the current image and the saved image into multiple regions;
a gradation-value analyzing portion that calculates histograms of gradation values of the regions divided by the region-dividing portion for the current image and the saved image; and
a gradation-value adjusting portion that adjusts gradation values of the individual regions in directions in which overlapping regions between the histograms of the current image calculated by the gradation-value analyzing portion and the histograms of the saved image are increased.

12. An endoscope apparatus according Claim 1, further comprising:
a region-dividing portion that divides the current image and the saved image into multiple regions; and
a gradation-value detecting portion that detects gradation values of the regions divided by the region-dividing portion for the current image and the saved image, wherein
in the case in which a saturated region in which the gradation values have saturated exists in the current image, the image combining portion replaces an image of the saturated region with the saved image.

13. An endoscope apparatus according to Claim 1, further comprising:
a characteristic-point detecting portion that detects a characteristic point in the current image; and
a characteristic-point searching portion that searches for the characteristic point detected by the characteristic-point detecting portion in a plurality of saved images saved in the image saving portion, wherein
the treatment-instrument-corresponding-region extracting portion extracts a region corresponding to the treatment-instrument region from the saved image in which the characteristic point has been searched for by the characteristic-point searching portion.

14. An endoscope apparatus according to Claim 1, wherein
the treatment-instrument-corresponding-region extracting portion enlarges or reduces the saved image and extracts the region corresponding to the treatment-instrument region from the enlarged or reduced saved image.

15. An endoscope apparatus according to Claim 14, further comprising:
a characteristic-point detecting portion that detects a plurality of characteristic points in the current image and the saved image; and
an enlargement-ratio setting portion that sets an enlargement ratio for the saved image relative to the current image on the basis of distances between the plurality of characteristic points in the current image and the saved image detected by the characteristic-point detecting portion,
wherein the treatment-instrument-corresponding-region extracting portion enlarges or reduces the saved image by the enlargement ratio set by the enlargement-ratio setting portion.
